(19) Europäisches Patentamt
European Patent Office
Office européen des brevets

(11) Veröffentlichungsnummer: **0 355 544 B1**

(12) **EUROPÄISCHE PATENTSCHRIFT**

(45) Veröffentlichungstag der Patentschrift: **26.01.94**

(51) Int. Cl.$^{5}$: **C07C 211/38**, A01N 33/04, C07C 217/08, C07D 307/14, C07D 317/28, C07D 295/033, C07D 265/30, C07D 295/073, C07D 211/14

(21) Anmeldenummer: **89114521.1**

(22) Anmeldetag: **05.08.89**

(54) **Substituierte Decalinamine, Verfahren zu deren Herstellung, sowie deren Verwendung in Schädlingsbekämpfungsmitteln.**

(30) Priorität: **19.08.88 DE 3828167**

(43) Veröffentlichungstag der Anmeldung:
**28.02.90 Patentblatt 90/09**

(45) Bekanntmachung des Hinweises auf die Patenterteilung:
**26.01.94 Patentblatt 94/04**

(84) Benannte Vertragsstaaten:
**BE CH DE FR GB IT LI NL**

(56) Entgegenhaltungen:
**EP-A- 0 254 150**
**EP-A- 0 278 311**
**EP-A- 0 309 913**
**EP-A- 0 319 914**

**SYNTHESIS, November 1987, Seiten 1005-1007; H.J. SCHAFER et al.: "Reductivea-mination of ketones and aldehydes at the mercury cathode"**

(73) Patentinhaber: **BAYER AG**

**D-51368 Leverkusen(DE)**

(72) Erfinder: **Weissmueller, Joachim, Dr.**
**Carl-Langhans-Strasse 53**
**D-4019 Monheim(DE)**
Erfinder: **Dutzmann, Stefan, Dr.**
**Leinenweberweg 33**
**D-4000 Dusseldorf 13(DE)**

**EP 0 355 544 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte Decalinamine, mehrere Verfahren zu ihrer Herstellung und ihre Verwendung in Schädlingsbekämpfungsmitteln.

Es ist bekannt, daß bestimmte Decalinylalkylaminoverbindungen, wie beispielsweise die Verbindung 2-[2-Methyl-3-(piperidin-1-yl)-propyl]-6-methyldecalin fungizide Eigenschaften besitzen (vergl. z.B. EP 254 150, EP- 309 913, EP- 309 914).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Weiterhin sind bestimmte Decalinamine, wie beispielsweise die Verbindungen 2-Aminodecalin, N-Methyl-2-aminodecalin, N-Ethyl-2-aminodecalin, N,N-Dimethyl-2-aminodecalin oder 2-(1-Pyrrolidinyl)-decalin bekannt (vergl. z.B. Synthesis 1987, 1005-1007; US 3 927 020; J. med. Chem. 15, 545-548 [1972]; J. org. Chem. 40, 1308-1312 [1975]; J. Am. chem. Soc. 85, 207-222 [1963]; Tetrahedron Lett. 1963, 825-830).

Die Wirksamkeit der vorbekannten Verbindungen gegenüber Schädlingen wird jedoch nicht beschrieben.

Es wurden neue substituierte Decalinamine der allgemeinen Formel (I),

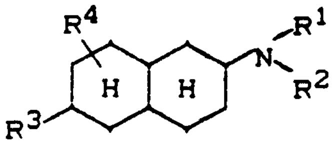
(I)

in welcher

| | |
|---|---|
| $R^1$ | für Alkyl mit mehr als zwei Kohlenstoffatomen, für Alkenyl, Alkinyl, Alkoxyalkyl, Dialkoxyalkyl, Hydroxyalkyl, Hydroxyalkoxyalkyl, Tetrahydrofuranylalkyl, Tetrahydropyranylalkyl, Dioxolanylalkyl, Dioxanylalkyl oder für jeweils unsubstituiertes oder substituiertes Cycloalkylalkyl, Cycloalkyl, Aralkyl, Aralkenyl oder Aryl steht, |
| $R^2$ | für Wasserstoff, Alkyl, Alkenyl, Alkinyl, Alkoxyalkyl, Dialkoxyalkyl, Hydroxyalkyl, Hydroxyalkoxyalkyl, Tetrahydrofuranylalkyl, Tetrahydropyranylalkyl, Dioxolanylalkyl, Dioxanylalkyl oder für jeweils unsubstituiertes oder substituiertes Cycloalkylalkyl, Cycloalkyl, Aralkyl, Aralkenyl oder Aryl steht oder |
| $R^1$ und $R^2$ | gemeinsam mit dem Stickstoffatom, an welches sie gebunden, sind für einen unsubstituierten oder substituierten gesättigten Heterocyclus stehen, der gegebenenfalls weitere Heteroatome enthalten kann und |
| $R^3$ und $R^4$ | unabhängig voneinander jeweils für Wasserstoff, Alkyl, Halogenalkyl, Alkoxy oder Halogenalkoxy stehen, |

wobei jedoch $R^1$ und $R^2$ gemeinsam mit dem Stickstoffatom, an welches sie gebunden sind, nur dann für einen unsubstituierten oder substituierten Pyrrolidinylrest stehen, wenn $R^3$ und $R^4$ nicht gleichzeitig für Wasserstoff stehen, und für den Fall, daß $R^4$ für 9-Methyl steht, $R^3$ nicht für Wasserstoff steht,
sowie deren Säureadditionssalze gefunden.

Die Verbindungen der Formel (I) können als geometrische und gegebenenfalls auch als optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Decalinamine der allgemeinen Formel (I),

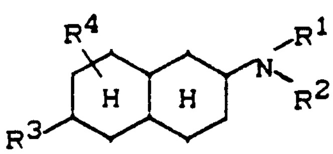
(I)

in welcher

| | |
|---|---|
| $R^1$ | für Alkyl mit mehr als zwei Kohlenstoffatomen, für Alkenyl, Alkinyl, Alkoxyalkyl, Dialkoxyalkyl, Hydroxyalkyl, Hydroxyalkoxyalkyl, Tetrahydrofuranylalkyl, Tetrahydropyranylalkyl, Dioxolanylalkyl, Dioxanylalkyl oder für jeweils unsubstituiertes oder substituiertes Cycloalkylal- |

kyl, Cycloalkyl, Aralkyl, Aralkenyl oder Aryl steht,

$R^2$ für Wasserstoff, Alkyl, Alkenyl, Alkinyl, Alkoxyalkyl, Dialkoxyalkyl, Hydroxyalkyl, Hydroxyalkoxyalkyl, Tetrahydrofuranylalkyl, Tetrahydropyranylalkyl, Dioxolanylalkyl, Dioxanylalkyl oder für jeweils unsubstituiertes oder substituiertes Cycloalkylalkyl, Cycloalkyl, Aralkyl, Aralkenyl oder Aryl steht oder

$R^1$ und $R^2$ gemeinsam mit dem Stickstoffatom, an welches sie gebunden sind, für einen unsubstituierten oder substituierten gesättigten Heterocyclus stehen, der gegebenenfalls weitere Heteroatome enthalten kann und

$R^3$ und $R^4$ unabhängig voneinander jeweils für Wasserstoff, Alkyl, Halogenalkyl, Alkoxy oder Halogenalkoxy stehen,

wobei jedoch $R^1$ und $R^2$ gemeinsam mit dem Stickstoffatom, an welches sie gebunden sind, nur dann für einen unsubstituierten oder substituierten Pyrrolidinylrest stehen, wenn $R^3$ und $R^4$ nicht gleichzeitig für Wasserstoff stehen, und für den Fall, daß $R^4$ für 9-Methyl steht, $R^3$ nicht für Wasserstoff steht,
sowie deren Säureadditionssalze erhält, wenn man

(a) Decalinone der Formel (II),

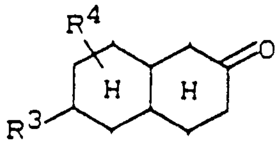

(II)

in welcher

$R^3$ und $R^4$ die oben angegebene Bedeutung haben,

mit Aminen der Formel (III),

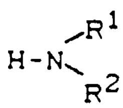

(III)

in welcher

$R^1$ und $R^2$ die oben angegebene Bedeutung haben,

in Gegenwart eines Reduktionsmittels, gegebenenfalls in Gegenwart eines Verdünnungsmittels und gegebenenfalls in Gegenwart eines Reaktionshilfsmittels umsetzt oder wenn man

b) substituierte ß-Naphthylamine der Formel (IV),

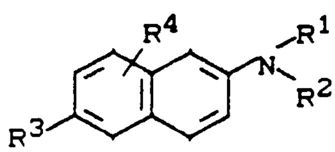

(IV)

in welcher

$R^1$, $R^2$, $R^3$ und $R^4$ die oben angegebene Bedeutung haben,

mit Wasserstoff in Gegenwart eines Katalysators und gegebenenfalls in Gegenwart eines Verdünnungsmittels hydriert;

desweiteren erhält man substituierte Decalinamine der Formel (Ia),

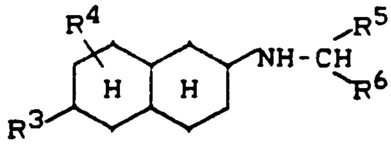

(Ia)

in welcher

$R^5$ für Alkyl, Alkenyl, Alkinyl, Alkoxy, Alkoxyalkyl, Dialkoxyalkyl, Hydroxyalkyl, Hydroxyalkoxyalkyl, Tetrahydrofuranyl, Tetrahydropyranyl, Dioxolanyl, Dioxanyl, für Tetrahydrofuranylalkyl, Tetrahydropyranylalkyl, Dioxolanylalkyl, Dioxanylalkyl oder für jeweils unsubstituiertes oder substituiertes Cycloalkylalkyl, Cycloalkyl, Aralkyl, Aralkenyl oder Aryl steht,

$R^6$ für Wasserstoff oder Alkyl steht und

$R^3$ und $R^4$ die oben angegebene Bedeutung haben,

wobei $R^5$ nur dann für Methyl steht, wenn $R^6$ nicht gleichzeitig für Wasserstoff steht,

alternativ auch, wenn man

(c) Decalinamine der Formel (V),

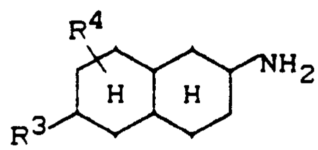

(V)

in welcher

$R^3$ und $R^4$ die oben angegebene Bedeutung haben,

mit Aldehyden oder Ketonen der Formel (VI),

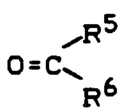

(VI)

in welcher

$R^5$ und $R^6$ die oben angegebene Bedeutung haben,

in Gegenwart eines Reduktionsmittels, gegebenenfalls in Gegenwart eines Verdünnungsmittels und gegebenenfalls in Gegenwart eines Reaktionshilfsmittels umsetzt;

ferner erhält man substituierte Decalinamine der Formel (Ib),

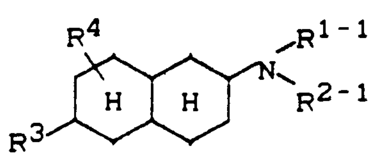

(Ib)

in welcher

$R^{1-1}$ für Alkyl mit mehr als zwei Kohlenstoffatomen, für Alkenyl, Alkinyl, Alkoxyalkyl, Dialkoxyalkyl, Hydroxyalkyl, Hydroxyalkoxyalkyl, Tetrahydrofuranylalkyl, Tetrahydropyranylalkyl, Dioxolanylalkyl, Dioxanylalkyl, für jeweils unsubstituiertes oder substituiertes Cycloalkylalkyl, Cycloalkyl, Aralkyl oder Aralkenyl steht,

$R^{2-1}$ für Wasserstoff, Alkyl, Alkenyl, Alkinyl, Alkoxyalkyl, Dialkoxyalkyl, Hydroxyalkyl, Hydroxyalkoxyalkyl, Tetrahydrofuranylalkyl, Tetrahydropyranylalkyl, Dioxolanylalkyl, Dioxanylalkyl oder für jeweils unsubstituiertes oder substiutiertes Cycloalkylalkyl, Cycloalkyl, Aralkyl, Aralkenyl oder Aryl steht und

$R^3$ und $R^4$ die oben angegebene Bedeutung haben,
alternativ auch, wenn man
(d) Decalinamine der Formel (VII),

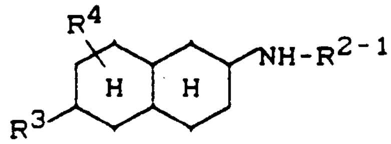

(VII)

in welcher
$R^{2-1}$, $R^3$ und $R^4$ die oben angegebene Bedeutung haben,
mit Alkylierungsmitteln der Formel (VIII),

$R^{1-1}$ - E (VIII)

in welcher
$R^{1-1}$ die oben angegebene Bedeutung hat und
E für eine elektronenanziehende Abgangsgruppe steht,
gegebenenfalls in Gegenwart eines Verdünnungsmittels und gegebenenfalls in Gegenwart eines Reaktionshilfsmittels umsetzt
und gegbenenfalls anschließend eine Säure addiert.

Schließlich wurde gefunden, daß die neuen substituierten Decalinamine der allgemeinen Formel (I) und deren Säureadditionssalze ausgezeichnete Wirksamkeit gegen Schädlinge besitzen.

Überraschenderweise zeigen die erfindungsgemäßen substituierten Decalinamine der allgemeinen Formel (I) eine deutlich bessere fungizide Wirksamkeit als die aus dem Stand der Technik bekannten wirkungsmäßig naheliegenden Decalinylalkylaminoverbindungen.

Die erfindungsgemäßen substituierten Decalinamine sind durch die Formel (I) allgemein definiert.

Alkyl steht im weiteren, falls nicht anders definiert für geradkettiges oder verzweigtes Alkyl mit 1 bis 12, vorzugsweise 1 bis 8 und insbesondere 1 bis 6 Kohlenstoffatomen sowie als Substituent in alkylhaltigen Resten für geradkettiges oder verzweigtes Alkyl mit 1 bis 8, vorzugsweise 1 bis 6 und insbesondere 1 bis 4 Kohlenstoffatomen. Beispielhaft seien genannt Methyl, Ethyl, n- und i-Propyl, n-, i-, s- und t-Butyl, Pentyl und Hexyl.

Alkenyl bzw. Alkinyl steht im weiteren, falls nicht anders definiert, für geradkettiges oder verzweigtes Alkenyl bzw. Alkinyl mit 3 bis 8, insbesondere 3 bis 5 Kohlenstoffatomen. Beispielhaft genannt seien Allyl, Butenyl, Pentenyl, Propargyl und Butinyl.

Cycloalkyl bzw. Cycloalkylalkyl steht im weiteren, falls nicht anders definiert für Verbindungen mit 3 bis 7, insbesondere 4 bis 6 Kohlenstoffatomen im Cycloalkylteil. Beispielhaft genannt seien: Cyclopropyl, Cyclopropylmethyl, Cyclopropylethyl, Cyclopropylpropyl, Cyclopentyl, Cyclopentylmethyl, Cyclohexyl und Cyclohexylmethyl.

Aryl steht im weiteren, auch in Aralkyl und Aralkenyl, falls nicht anders definiert, für Aryl mit 6 bis 10 Kohlenstoffatomen im Arylteil. Beispielhaft genannt seien Phenyl, Benzyl und Phenethyl.

Der gemeinsam von $R^1$, $R^2$ und dem Stickstoffatom gebildete Heterocyclus ist z.B. ein Rest der Formel

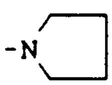 ; 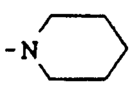 ; 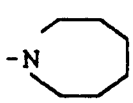 ;

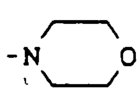 oder 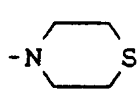

Halogen steht im weiteren für Fluor, Chlor, Brom und Iod vorzugsweise für Fluor und Chlor.

Die Reste der allgemeinen Formeln können gegebenenfalls einen oder mehrere, vorzugsweise 1 bis 5, insbesondere 1 bis 3 gleiche oder verschiedene Substituenten tragen. Als Substituenten seien beispielhaft und vorzugsweise aufgeführt:

Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 9, insbesondere 1 bis 5 Halogenatomen, wobei die Halogenatome gleich oder verschieden sind und als Halogenatome, vorzugsweise Fluor, Chlor oder Brom, insbesondere Fluor stehen, wie Trifluormethyl; Hydroxy; Halogen, vorzugsweise Fluor, Chlor, Brom und Jod, insbesondere Fluor, Chlor und Brom; Cyano; Nitro.

Bevorzugt sind Verbindungen der Formel (I), in denen

$R^1$ für jeweils geradkettiges oder verzweigtes Alkyl mit 3 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 8 Kohlenstoffatomen oder Hydroxyalkoxyalkyl mit 2 bis 6 Kohlenstoffatomen in den einzelnen Alkylteilen, für jeweils geradkettiges oder verzweigtes Tetrahydrofuranylalkyl, Tetrahydropyranylalkyl, Dioxolanylalkyl oder Dioxanylalkyl mit jeweils 1 bis 4 Kohlenstoffatomen im Alkylteil oder für jeweils unsubstituiertes oder im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und 1 bis 4 Kohlenstoffatomen im geradkettigen oder verzweigten Alkylteil steht, wobei als Substituenten jeweils infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; oder für jeweils unsubstituiertes oder einfach bis mehrfach, gleich oder verschieden im Arylteil substituiertes Arylalkyl, Arylalkenyl oder Aryl mit jeweils 6 bis 10 Kohlenstoffatomen im Arylteil und bis zu 6 Kohlenstoffatomen im geradkettigen oder verzweigten Alkyl- bzw. Alkenylteil steht, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen,

$R^2$ für Wasserstoff, für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 8 Kohlenstoffatomen oder Hydroxyalkoxyalkyl mit 2 bis 6 Kohlenstoffatomen in den einzelnen Alkylteilen, für jeweils geradkettiges oder verzweigtes Tetrahydrofuranylalkyl, Tetrahydropyranylalkyl, Dioxolanylalkyl oder Dioxanylalkyl mit jeweils 1 bis 4 Kohlenstoffatomen im Alkylteil oder für jeweils unsubstituiertes oder im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und 1 bis 4 Kohlenstoffatomen im geradkettigen oder verzweigten Alkylteil steht, wobei als Substituenten jeweils infrage kommen: jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;
oder für jeweils unsubstituiertes oder einfach bis mehrfach, gleich oder verschieden im Arylteil substituiertes Arylalkyl, Arylalkenyl oder Aryl mit jeweils 6 bis 10 Kohlenstoffatomen im Arylteil und gegebenenfalls bis zu 6 Kohlenstoffatomen im geradkettigen oder verzweigten Alkyl- bzw. Alkenylteil steht, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils jeweils 1 bis 4 Kohlenstoffatomen in den einzelnen Alkylteilen und gegebenenfalls 1 bis 9 gleichen oder verschiedenen Halogenatomen oder

$R^1$ und $R^2$ gemeinsam mit dem Stickstoffatom, an welches sie gebunden sind, für einen unsubstituierten oder einfach bis mehrfach, gleich oder verschieden substituierten, gesättigten 5- bis 7-gliedrigen Heterocyclus stehen, der gegebenenfalls ein weiteres Heteroatom, insbesondere Stickstoff, Sauerstoff oder Schwefel enthalten kann, wobei als Substituenten infrage kommen: jeweils geradkettiges oder verzweigtes Alkyl oder Hydroxyalkyl mit jeweils 1 bis 4 Kohlenstoffatomen und

$R^3$ und $R^4$ unabhängig voneinander jeweils für Wasserstoff, für jeweils geradkettiges oder verzweigtes Alkyl oder Alkoxy mit jeweils 1 bis 4 Kohlenstoffatomen oder für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen stehen,
wobei jedoch $R^1$ und $R^2$ gemeinsam mit dem Stickstoffatomen, an welches sie gebunden sind, nur dann für einen unsubstituierten oder substituierten Pyrrolidinylrest stehen, wenn $R^3$ und $R^4$ nicht gleichzeitig für Wasserstoff stehen.

Besonders bevorzugt sind Verbindungen der Formel (I), in denen

$R^1$ für jeweils geradkettiges oder verzweigtes Propyl, Butyl, Pentyl, Hexyl, Heptyl, Octyl, Allyl, Butenyl, Pentenyl, Propargyl, Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, für Tetrahydrofuranylmethyl, Tetrahydropyranylmethyl, Dioxolanylmethyl, Dioxolanylethyl, Dioxanylmethyl oder Dioxanylethyl, für jeweils unsubstituiertes oder ein- bis fünffach, gleich oder verschieden durch Methyl, Trifluormethyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl, Cyclohexylmethyl, Cyclohexylethyl, oder Cyclohexylpropyl oder für jeweils unsubstituiertes oder ein- bis dreifach, gleich oder verschieden substituiertes Phenyl, Benzyl oder Phenethyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl und

$R^2$ für Wasserstoff, Methyl, Ethyl, für jeweils geradkettiges oder verzweigtes Propyl, Butyl, Pentyl, Hexyl, Heptyl, Octyl, Allyl, Butenyl, Pentenyl, Propargyl, Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, für Tetrahydrofuranylmethyl, Tetrahydropyranylmethyl, Dioxolanylmethyl, Dioxolanylethyl, Dioxanylmethyl oder Dioxanylethyl, für jeweils unsubstituiertes oder ein- bis fünffach, gleich oder verschieden durch Methyl, Trifluormethyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils unsubstituiertes oder ein- bis dreifach, gleich oder verschieden substituiertes Phenyl, Benzyl, Phenylethyl, Phenylpropyl oder Phenylbutyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl oder

$R^1$ und $R^2$ gemeinsam mit dem Stickstoffatom, an welches sie gebunden sind, für einen unsubstituierten oder ein- bis dreifach, gleich oder verschieden substituierten Heterocyclus der Formel

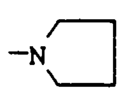 ; 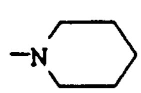 ; 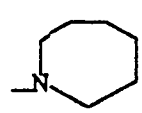 ; 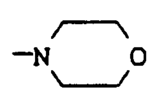

oder 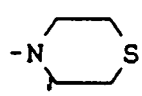

stehen, wobei als Substituenten infrage kommen: Methyl, Ethyl oder Hydroxymethyl und

$R^3$ und $R^4$ unabhängig voneinander jeweils für Wasserstoff, Methyl, Ethyl, n- oder i-Propyl, n-, i-, s- oder t-Butyl, für Trifluormethyl, Difluormethyl, Fluormethyl, Chlordifluormethyl, Dichlorfluormethyl, für Methoxy, Ethoxy, n- oder i-Propoxy, Trifluormethoxy oder Difluormethoxy stehen,

wobei jedoch $R^1$ und $R^2$ gemeinsam mit dem Stickstoffatom, an welches sie gebunden sind, nur dann für einen unsubstituierten oder substituierten Pyrrolidinylrest stehen, wenn $R^3$ und $R^4$ nicht gleichzeitig für Wasserstoff stehen.

Ganz besonders bevorzugt sind Verbindungen der Formel (I), in denen

$R^1$ für jeweils geradkettiges oder verzweigtes Propyl, Butyl, Pentyl, Hexyl, Allyl, Butenyl, Pentenyl, Propargyl, Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Tetrahydrofuranylmethyl, Tetrahydropyranylmethyl, Dioxolanylmethyl, Dioxolanylethyl, Dioxanylmethyl, Cyclopropylmethyl, Dichlorcyclopropylmethyl, Dimethylcyclopropylmethyl, Dichlordimethylcyclopropylmethyl, Cyclopentyl, Cyclohexyl oder Cyclohexylmethyl steht und

$R^2$ für Wasserstoff, Methyl, Ethyl oder für jeweils gegebenenfalls geradkettiges oder verzweigtes Propyl, Butyl, Pentyl, Hexyl, Allyl, Butenyl, Pentenyl, Propargyl, Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Tetrahydrofuranylmethyl, Tetrahydropyranylmethyl, Dioxolanylmethyl, Dioxolanylethyl, Dioxanylmethyl, Dioxanylethyl, Cyclopropylmethyl, Dimethylcyclopropylmethyl, Cyclopentyl, für jeweils unsubstituiertes oder einfach bis dreifach, gleich oder verschieden durch Methyl oder t-Butyl substituiertes Cyclohexyl, Cyclohexylmethyl, Benzyl, Phenylethyl oder Phenylpropyl steht oder

$R^1$ und $R^2$ gemeinsam mit dem Stickstoffatom, an welches sie gebunden sind, für einen unsubstituierten oder ein- bis dreifach, gleich oder verschieden substituierten Heterocyclus der Formel

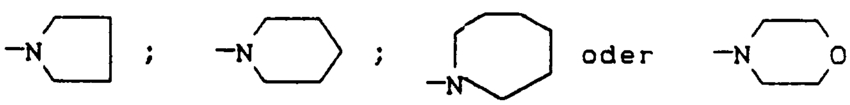

stehen

wobei als Substituenten jeweils infrage kommen: Methyl, Ethyl oder Hydroxymethyl und

$R^3$ und $R^4$ unabhängig voneinander jeweils für Wasserstoff, Methyl, Ethyl, n- oder i-Propyl, t-Butyl oder Trifluormethyl stehen,

wobei jedoch $R^1$ und $R^2$ gemeinsam mit dem Stickstoffatom, an welches sie gebunden sind, nur dann für einen unsubstituierten oder substituierten Pyrrolidinylrest stehen, wenn $R^3$ und $R^4$ nicht gleichzeitig für Wasserstoff stehen.

Bevorzugte erfindungsgemäße Verbindungen sind auch Additionsprodukte insbesondere die pflanzenverträglichen Additionsprodukte aus Säuren und denjenigen substituierten Decalinaminen der Formel (I), in denen die Substituenten $R^1$, $R^2$, $R^3$ und $R^4$ die Bedeutungen haben, die bereits vorzugsweise für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Im einzelnen seien außer den bei den Herstellungsbeispielen genannten Verbindungen die folgenden substituierten Decalinamine der allgemeinen Formel (I) genannt:

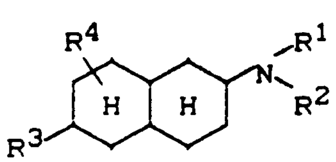

(I)

| 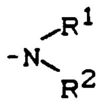 | $R^3$ | $R^4$ |
|---|---|---|
| $-NH-(CH_3)_2-CH_3$ | H | H |
| $-NH-CH_2-CH(CH_3)_2$ | H | H |
| $-NH-CH_2-CH(C_2H_5)_2$ | H | H |
| $-NH-(CH_2)_5-CH_3$ | H | H |
| $-NH-(CH_2)_7-CH_3$ | H | H |
| 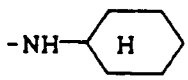 | H | H |
| 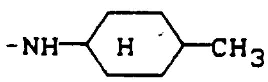 | H | H |
| 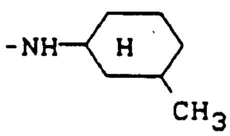 | H | H |
| $-NH-CH_2-CH_2-CH_3$ | H | H |
| $-N(CH(CH_3)_2)-(CH_2)_3-CH_3$ | H | H |
| $-N(CH(CH_3)_2)-CH_2-CH(CH_3)_2$ | H | H |
| $-N(CH(CH_3)_2)-C(CH_3)_3$ | H | H |
| $-N(CH(CH_3)_2)-CH(CH_3)_2$ | H | H |
| $-N(CH(CH_3)_2)-CH_2-CH(C_2H_5)_2$ | H | H |
| $-N(CH(CH_3)_2)-(CH_2)_5-CH_3$ | H | H |

| 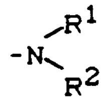 | $R^3$ | $R^4$ |
|---|---|---|
| 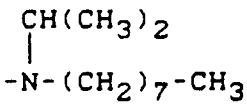 | H | H |
| 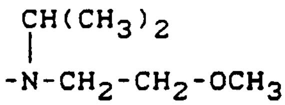 | H | H |
| $-NH-CH_2-CH_2-CH_2-OC_2H_5$ | H | H |
| 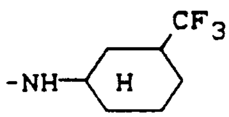 | H | H |
| 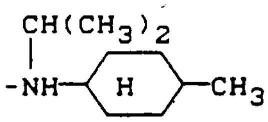 | H | H |
| 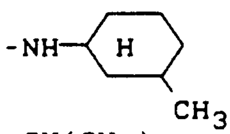 | H | H |
| 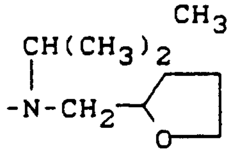 | H | H |
| 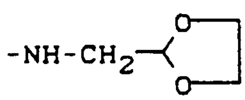 | H | H |
| 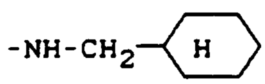 | H | H |
| 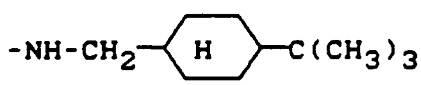 | H | H |
| 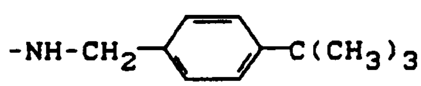 | H | H |
| 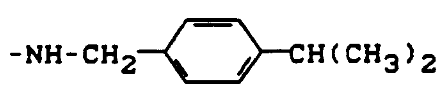 | H | H |
| 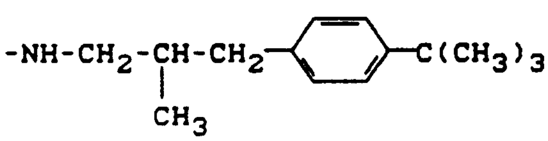 | H | H |

| 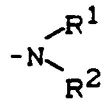 | $R^3$ | $R^4$ |
|---|---|---|
| 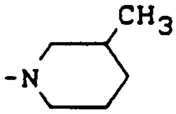 | $-CH(CH_3)_2$ | H |
| 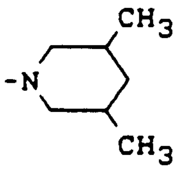 | $-CH(CH_3)_2$ | H |
| 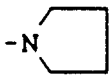 | H | H |
| 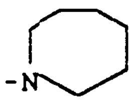 | H | H |
| 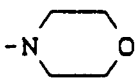 | H | H |
| 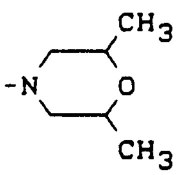 | $-CH(CH_3)_2$ | H |
| 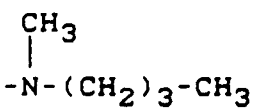 | H | H |
| 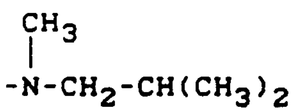 | H | H |
| 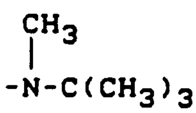 | H | H |
| 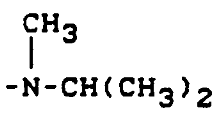 | H | H |

| 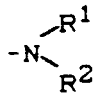 | $R^3$ | $R^4$ |
|---|---|---|
| 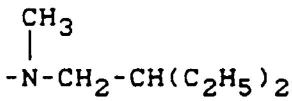 | H | H |
| 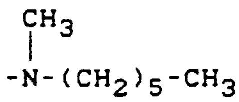 | H | H |
| 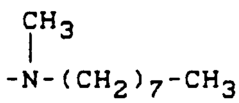 | H | H |
| 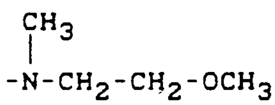 | H | H |
| 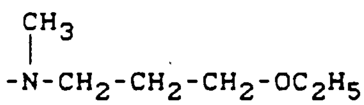 | H | H |
| 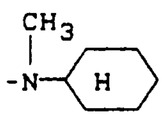 | H | H |
| 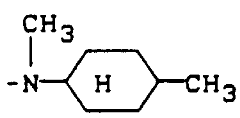 | H | H |
| 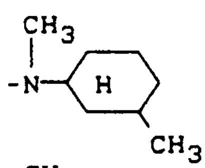 | H | H |
| 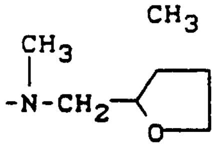 | H | H |
| 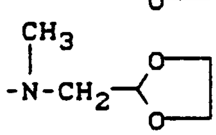 | H | H |
| 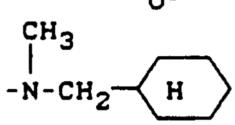 | H | H |
| 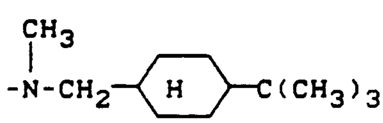 | H | H |

| 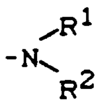 | $R^3$ | $R^4$ |
|---|---|---|
| 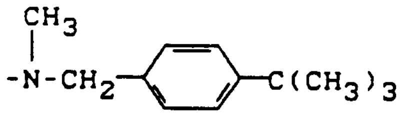 | H | H |
| 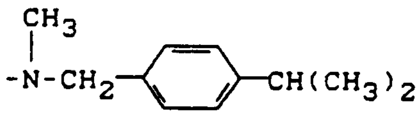 | H | H |
| 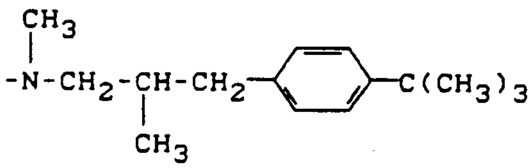 | H | H |
| 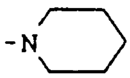 | $-CH(CH_3)_2$ | H |
| 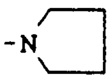 | $-CH(CH_3)_2$ | H |
| 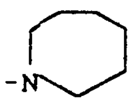 | $-CH(CH_3)_2$ | H |
| 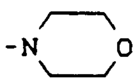 | $-CH(CH_3)_2$ | H |
| 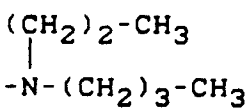 | H | H |
| 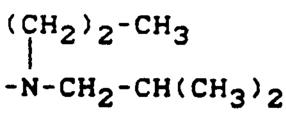 | H | H |
| 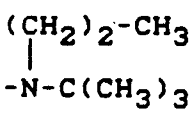 | H | H |
| 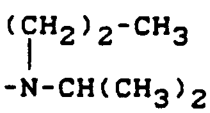 | H | H |
| 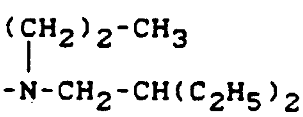 | H | H |

| 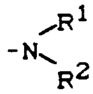 | $R^3$ | $R^4$ |
|---|---|---|
| 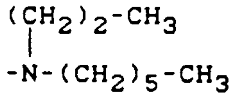 | H | H |
| 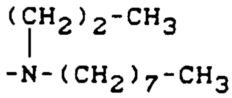 | H | H |
| 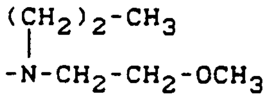 | H | H |
| 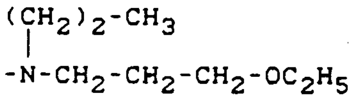 | H | H |
| 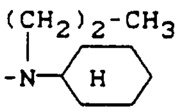 | H | H |
| 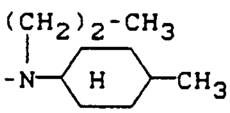 | H | H |
| 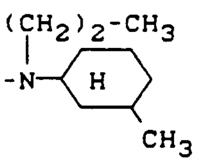 | H | H |
| 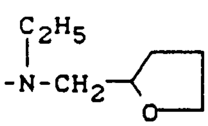 | H | H |
| 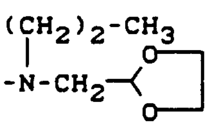 | H | H |
| 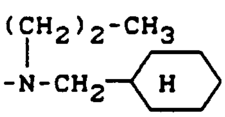 | H | H |
| 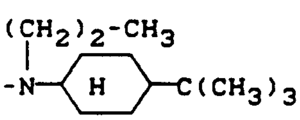 | H | H |

| 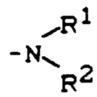 | $R^3$ | $R^4$ |
|---|---|---|
| 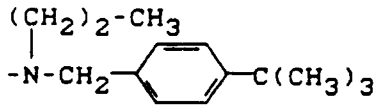 | H | H |
| 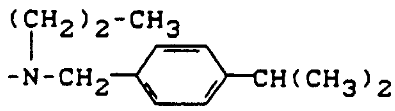 | H | H |
| 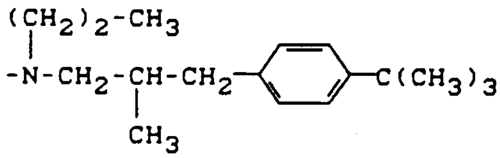 | H | H |
| 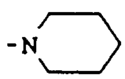 | $CF_3$ | H |
| 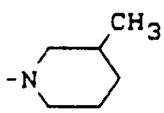 | $CF_3$ | H |
| 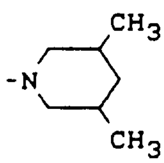 | $CF_3$ | H |
| 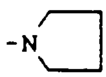 | $CF_3$ | H |
| 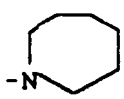 | $CF_3$ | H |
| 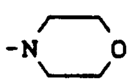 | $CF_3$ | H |
| 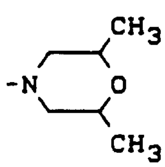 | $CF_3$ | H |
| 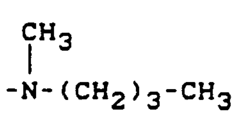 | $CH_3$ | H |

| 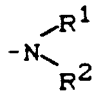 | $R^3$ | $R^4$ |
|---|---|---|
| $CH_3$<br>\|<br>$-N-CH_2-CH(CH_3)_2$ | $CH_3$ | H |
| $CH_3$<br>\|<br>$-N-C(CH_3)_3$ | $CH_3$ | H |
| $CH_3$<br>\|<br>$-N-CH(CH_3)_2$ | $CH_3$ | H |
| $CH_3$<br>\|<br>$-N-CH_2-CH(C_2H_5)_2$ | $CH_3$ | H |
| $CH_3$<br>\|<br>$-N-(CH_2)_5-CH_3$ | $CH_3$ | H |
| $CH_3$<br>\|<br>$-N-(CH_2)_7-CH_3$ | $CH_3$ | H |
| $CH_3$<br>\|<br>$-N-CH_2-CH_2-OCH_3$ | $CH_3$ | H |
| $CH_3$<br>\|<br>$-N-CH_2-CH_2-CH_2-OC_2H_5$ | $CH_3$ | H |
| 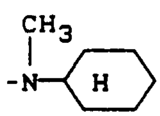 | $CH_3$ | H |
| 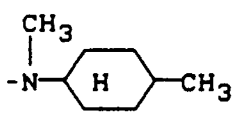 | $CH_3$ | H |
| 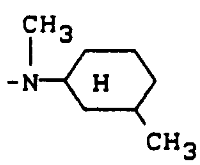 | $CH_3$ | H |

| 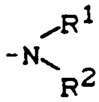 | $R^3$ | $R^4$ |
|---|---|---|
| 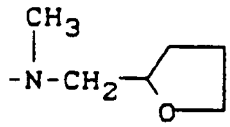 | $CH_3$ | H |
| 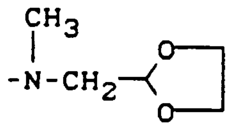 | $CH_3$ | H |
| 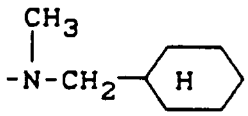 | $CH_3$ | H |
| 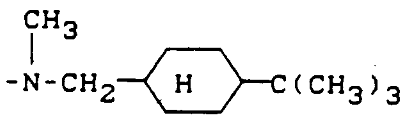 | $CH_3$ | H |
| 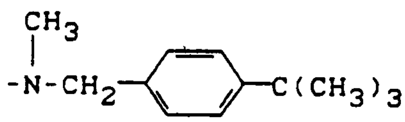 | $CH_3$ | H |
| 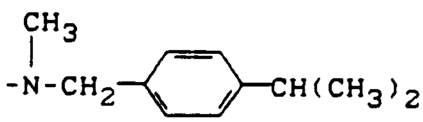 | $CH_3$ | H |
| 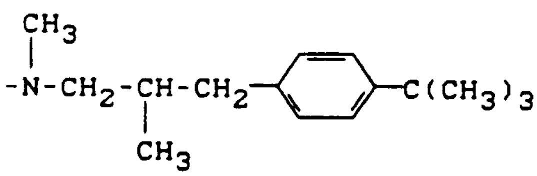 | $CH_3$ | H |
| 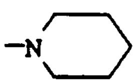 | $CH_3$ | H |
| 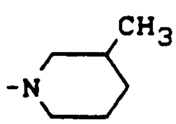 | $CH_3$ | H |
| 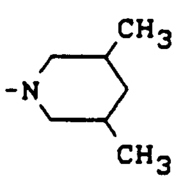 | $CH_3$ | H |
| 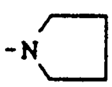 | $CH_3$ | H |

| 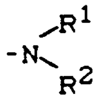 | $R^3$ | $R^4$ |
|---|---|---|
| 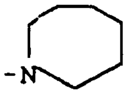 | $CH_3$ | H |
| 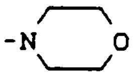 | $CH_3$ | H |
| $(CH_2)_2-CH_3$<br>\|<br>$-N-(CH_2)_3-CH_3$ | $CH_3$ | H |
| $(CH_2)_2-CH_3$<br>\|<br>$-N-CH_2-CH(CH_3)_2$ | $CH_3$ | H |
| $(CH_2)_2-CH_3$<br>\|<br>$-N-C(CH_3)_3$ | $CH_3$ | H |
| $(CH_2)_2-CH_3$<br>\|<br>$-N-CH(CH_3)_2$ | $CH_3$ | H |
| $(CH_2)_2-CH_3$<br>\|<br>$-N-CH_2-CH(C_2H_5)_2$ | $CH_3$ | H |
| $(CH_2)_2-CH_3$<br>\|<br>$-N-(CH_2)_5-CH_3$ | $CH_3$ | H |
| $(CH_2)_2-CH_3$<br>\|<br>$-N-(CH_2)_7-CH_3$ | $CH_3$ | H |
| $(CH_2)_2-CH_3$<br>\|<br>$-N-CH_2-CH_2-OCH_3$ | $CH_3$ | H |
| $(CH_2)_2-CH_3$<br>\|<br>$-N-CH_2-CH_2-CH_2-OC_2H_5$ | $CH_3$ | H |
| $(CH_2)_2-CH_3$<br>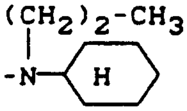 | $CH_3$ | H |

| 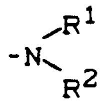 | $R^3$ | $R^4$ |
|---|---|---|
| 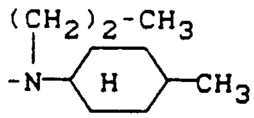 | $CH_3$ | H |
| 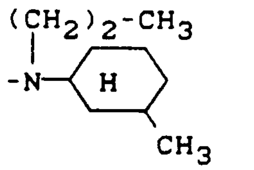 | $CH_3$ | H |
| 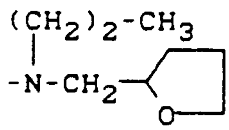 | $CH_3$ | H |
| 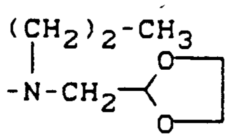 | $CH_3$ | H |
| 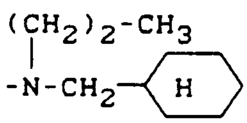 | $CH_3$ | H |
| 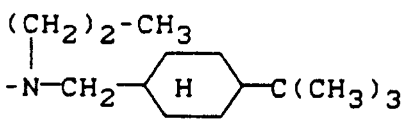 | $CH_3$ | H |
| 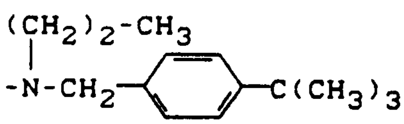 | $CH_3$ | H |
| 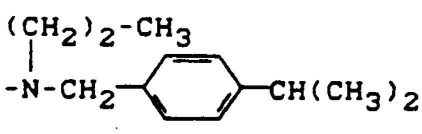 | $CH_3$ | H |
| 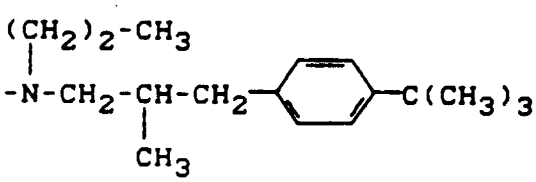 | $CH_3$ | H |
| 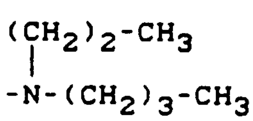 | $-C(CH_3)_3$ | H |

| 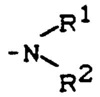 | $R^3$ | $R^4$ |
|---|---|---|
| $(CH_2)_2-CH_3$<br>\|<br>$-N-CH_2-CH(CH_3)_2$ | $-C(CH_3)_3$ | H |
| $(CH_2)_2-CH_3$<br>\|<br>$-N-C(CH_3)_3$ | $-C(CH_3)_3$ | H |
| $(CH_2)_2-CH_3$<br>\|<br>$-N-CH(CH_3)_2$ | $-C(CH_3)_3$ | H |
| $(CH_2)_2-CH_3$<br>\|<br>$-N-CH_2-CH(C_2H_5)_2$ | $-C(CH_3)_3$ | H |
| $(CH_2)_2-CH_3$<br>\|<br>$-N-(CH_2)_5-CH_3$ | $-C(CH_3)_3$ | H |
| $(CH_2)_2-CH_3$<br>\|<br>$-N-(CH_2)_7-CH_3$ | $-C(CH_3)_3$ | H |
| $(CH_2)_2-CH_3$<br>\|<br>$-N-CH_2-CH_2-OCH_3$ | $-C(CH_3)_3$ | H |
| $(CH_2)_2-CH_3$<br>\|<br>$-N-CH_2-CH_2-CH_2-OC_2H_5$ | $-C(CH_3)_3$ | H |
| 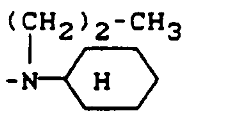 | $-C(CH_3)_3$ | H |
| 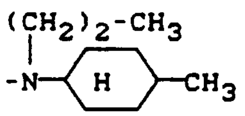 | $-C(CH_3)_3$ | H |
| 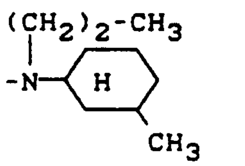 | $-C(CH_3)_3$ | H |

| 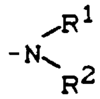 | $R^3$ | $R^4$ |
|---|---|---|
| 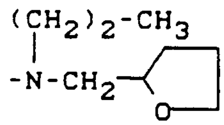 | $-C(CH_3)_3$ | H |
| 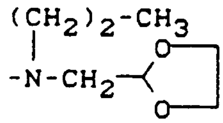 | $-C(CH_3)_3$ | H |
| 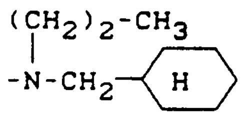 | $-C(CH_3)_3$ | H |
| 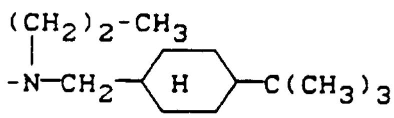 | $-C(CH_3)_3$ | H |
| 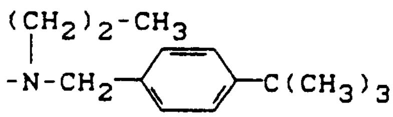 | $-C(CH_3)_3$ | H |
| 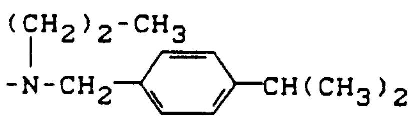 | $-C(CH_3)_3$ | H |
| 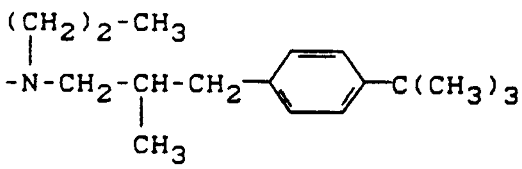 | $-C(CH_3)_3$ | H |
| 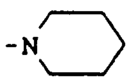 | $-C(CH_3)_3$ | H |
| 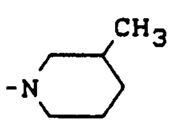 | $-C(CH_3)_3$ | H |
| 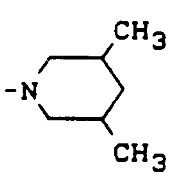 | $-C(CH_3)_3$ | H |
| 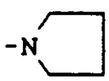 | $-C(CH_3)_3$ | H |

| 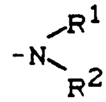 | $R^3$ | $R^4$ |
| --- | --- | --- |
| 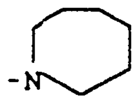 | $-C(CH_3)_3$ | H |
| 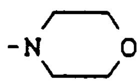 | $-C(CH_3)_3$ | H |
| 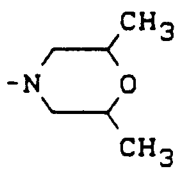 | $-C(CH_3)_3$ | H |

Verwendet man beispielsweise 2-Decalinon und Piperidin als Ausgangsstoffe sowie Natriumcyanoborhydrid als Reduktionsmittel, so läßt sich der Reaktionsablauf des erfindungsgemäßen Verfahrens (a) durch das folgende Formelschema darstellen:

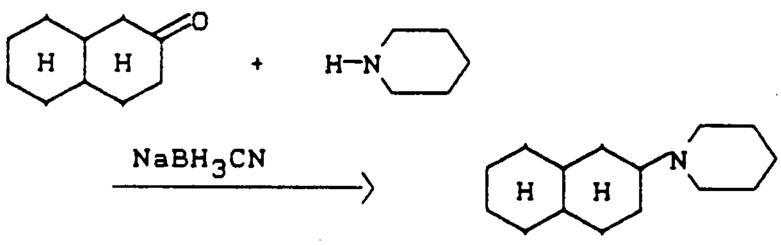


Verwendet man beispielsweise 2-(N-Isopropylamino)-naphthalin als Ausgangsverbindung und Ruthenium auf Aktivkohle als Katalysator, so läßt sich der Reaktionsablauf des erfindungsgemäßen Verfahrens (b) durch das folgende Formelschema darstellen:

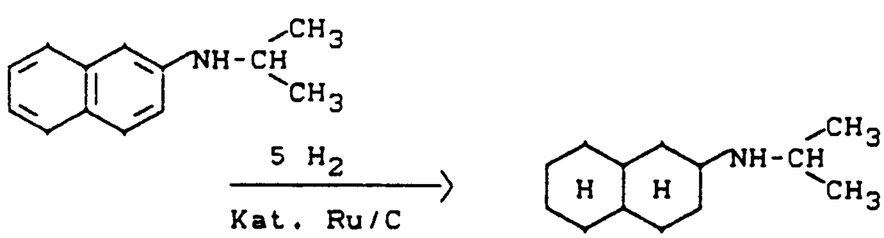


Verwendet man beispielsweise 2-Aminodecalin und Tetrahydrofuran-2-aldehyd als Ausgangsstoffe sowie molekularen Wasserstoff in Gegenwart von Palladium auf Aluminiumoxid als Reduktionsmittel, so läßt sich der Reaktionsablauf des erfindungsgemäßen Verfahrens (c) durch das folgende Formelschema darstellen:

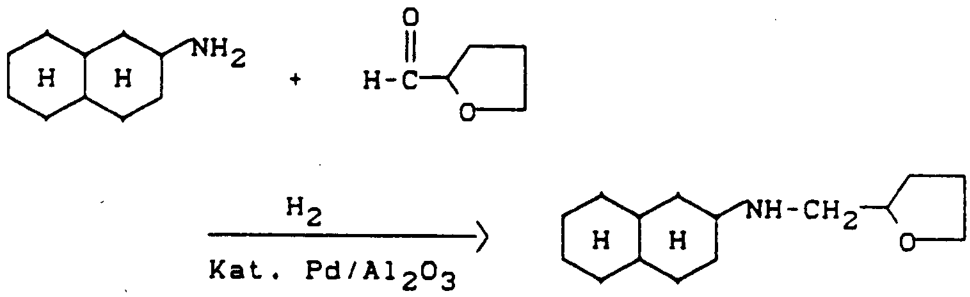


Verwendet man beispielsweise 2-(N-Methylamino)-decalin und 2-Tetrahydrofuranylmethylbromid als Ausgangsstoffe, so läßt sich der Reaktionsablauf des erfindungsgemäßen Verfahrens (d) durch das folgende Formelschema darstellen:

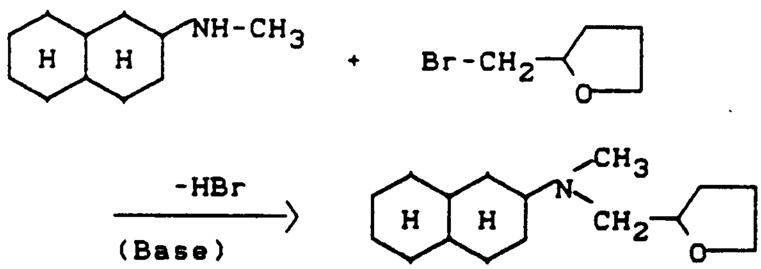


Die zur Durchführung des erfindungsgemäßen Verfahrens (a) als Ausgangsstoffe benötigten Decalinone sind durch die Formel (II) allgemein definiert. In dieser Formel (II) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die Decalinone der Formel (II) sind bekannt oder erhältlich in Analogie zu bekannten Verfahren (vergl. z.B. Arzneimittel Forsch. 13, 991-999 [1963]; Bull. Soc. Chim. France 1970, 2414-2415; Aust. J. Chem. 31, 803-813 [1978]; Tetrahedron 25, 5163-5175 [1969]).

Die zur Durchführung des erfindungsgemäßen Verfahrens (a) weiterhin als Ausgangsstoffe benötigten Amine sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind allgemein bekannte Verbindungen der organischen Chemie (vergl. z.B. Rocz. Chem. 42, 353 [1968]; JP 60/146 885).

Die zur Durchführung des erfindungsgemäßen Verfahrens (b) als Ausgangsstoffe benötigten ß-Naphthylamine sind durch die Formel (IV) allgemein definiert. In dieser Formel (IV) stehen $R^1$, $R^2$, $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die ß-Naphthylamine der Formel (IV) sind bekannt oder erhältlich in Analogie zu bekannten Verfahren (vergl. z.B. J. Amer. Chem. Soc. 78, 6265-6269 [1956] oder US 4 241 195).

Die zur Durchführung des erfindungsgemäßen Verfahrens (c) als Ausgangsstoffe benötigten Decalinamine sind durch die Formel (V) allgemein definiert. In dieser Formel (V) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die Decalinamine der Formel (V) sind bekannt oder erhältlich in Analogie zu bekannten Verfahren (vergl. z.B. J. Am. Chem. Soc. 91, 533-535 [1969]; J. Am. Chem. Soc. 90, 210-212 [1968]; US-PS 3 927 020).

Die zur Durchführung des erfindungsgemäßen Verfahrens (c) weiterhin als Ausgangsstoffe benötigten Aldehyde oder Ketone sind durch die Formel (VI) allgemein definiert. In dieser Formel (VI) steht

$R^5$ vorzugsweise für jeweils gradkettiges oder verzweigtes Alkyl mit 1 bis 10 Kohlenstoffatomen,

Alkenyl mit 2 bis 6 Kohlenstoffatomen, Alkinyl mit 2 bis 6 Kohlenstoffatomen, Alkoxy oder Hydroxyalkyl mit jeweils 1 bis 4 Kohlenstoffatomen, Alkoxyalkyl oder Dialkoxyalkyl mit jeweils 1 bis 4 Kohlenstoffatomen oder Hydroxyalkoxyalkyl mit 1 bis 4 Kohlenstoffatomen in den einzelnen Alkylteilen, für Tetrahydrofuranyl, Tetrahydropyranyl, Dioxolanyl, Dioxanyl, für Tetrahydrofuranylalkyl, Tetrahydropyranylalkyl, Dioxolanylalkyl oder Dioxanylalkyl mit jeweils 1 bis 2 Kohlenstoffatomen im geradkettigen oder verzweigten Alkylteil oder für jeweils unsubstituiertes oder im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und 1 bis 2 Kohlenstoffatomen im geradkettigen oder verzweigten Alkylteil, wobei als Substituenten infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;

$R^5$ steht außerdem für jeweils unsubstituiertes oder einfach bis mehrfach, gleich oder verschieden im Arylteil substituiertes Arylalkyl, Arylalkenyl oder Aryl mit jeweils 6 bis 10 Kohlenstoffatomen im Arylteil und bis zu 4 Kohlenstoffatomen im geradkettigen oder verzweigten Alkyl- bzw. Alkenylteil, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen und

$R^6$ steht vorzugsweise für Wasserstoff oder für geradkettiges oder verzweigtes Alkyl mit 1 bis 10 Kohlenstoffatomen,

wobei $R^5$ nur dann für Methyl steht, wenn $R^6$ nicht gleichzeitig für Wasserstoff steht.

$R^5$ steht besonders bevorzugt für Methyl, Ethyl, für jeweils geradkettiges oder verzweigtes Propyl, Butyl, Pentyl, Hexyl, Vinyl, Allyl, Butenyl, Ethinyl, Propargyl, Butinyl, Methoxy, Ethoxy, Propoxy, Hydroxymethyl, Hydroxyethyl, Hydroxy-Propyl, Methoxymethyl, Ethoxymethyl, Methoxyethyl, Ethoxyethyl, Propoxymethyl, Propoxyethyl, Butoxymethyl, Butoxyethyl, Hydroxyethoxymethyl, Dimethoxymethyl, Dimethoxyethyl, Diethoxyethyl, Tetrahydrofuranyl, Tetrahydropyranyl, Dioxolanyl, Dioxanyl, Tetrahydrofuranylmethyl, Tetrahydrofuranylethyl, Tetrahydropyranylmethyl, Tetrahydropyranylethyl, Dioxolanylmethyl, Dioxolanylethyl, Dioxanylmethyl, Dioxanylethyl, für jeweils unsubstituiertes oder ein- bis fünffach, gleich oder verschieden durch Methyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl, Cyclohexylmethyl, Cyclohexylethyl oder Cyclohexylpropyl oder für jeweils unsubstituiertes oder ein- bis dreifach, gleich oder verschieden substituiertes Phenyl, Benzyl, Phenylethyl, Phenylpropyl oder Phenylbutyl, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl und

$R^6$ steht vorzugsweise für Wasserstoff, Methyl, Ethyl, für jeweils geradkettiges oder verzweigtes Propyl, Butyl, Pentyl oder Hexyl,

wobei $R^5$ nur dann für Methyl steht, wenn $R^6$ nicht gleichzeitig für Wasserstoff steht.

Die Aldehyde oder Ketone der Formel (VI) sind bekannt oder erhältlich in Analogie zu bekannten Verfahren (vergl. z.B. J. Heterocycl. Chem. 18, 565-569 [1981]; Tetrahedron 36, 1649-1665 [1980], J. Amer. Chem. Soc. 95, 3635-3640 [1973]; Khim. Geterotsikl. Soedin. 1968, 359 bzw. CA 69: 106 362j; Agricult. Biol. Chem. 48, 2135-2136 [1984]; Helv. Chim. Acta 56, 3056-3059 [1973]).

Die zur Durchführung des erfindungsgemäßen Verfahrens (d) als Ausgangsstoffe benötigten Decalinamine sind durch die Formel (VII) allgemein definiert. In dieser Formel (VII) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

$R^{2-1}$ steht vorzugsweise für Wasserstoff, für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für jeweils geradkettiges oder verzweigtes Tetrahydrofuranylalkyl, Tetrahydropyranylalkyl, Dioxolanylalkyl oder Dioxanylalkyl mit jeweils 1 bis 4 Kohlenstoffatomen im Alkylteil oder für jeweils unsubstituiertes oder im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und 1 bis 4 Kohlenstoffatomen im geradkettigen oder verzweigten Alkylteil, wobei als Substituenten infrage kommen:
Halogen, jeweils geradkettiges oder verzweigtes Alkyl, Alkoxy, Halogenalkyl oder Halogenalkoxy mit jeweils

1 bis 4 Kohlenstoffatomen und gegebenenfalls 1 bis 9 gleichen oder verschiedenen Halogenatomen;
$R^{2-1}$ steht außerdem für jeweils unsubstituiertes oder einfach bis mehrfach, gleich oder verschieden im Arylteil substituiertes Arylalkyl, Arylalkenyl oder Aryl mit jeweils 6 bis 10 Kohlenstoffatomen im geradkettigen oder verzweigten Alkyl- bzw. Alkenylteil, wobei als Arylsubstituenten infrage kommen:
Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen.

$R^{2-1}$ steht besonders bevorzugt für Wasserstoff, Methyl, Ethyl, für jeweils geradkettiges oder verzweigtes Propyl, Butyl, Pentyl, Hexyl, Heptyl, Octyl, Allyl, Butenyl, Pentenyl, Propargyl, Butinyl, Hydroxyethyl, Hydroxypropyl, Methoxyethyl, Ethoxyethyl, Propoxyethyl, Butoxyethyl, Methoxypropyl, Propoxypropyl, Butoxypropyl, Hydroxyethoxyethyl, Dimethoxyethyl, Dimethoxypropyl, Diethoxyethyl, für Tetrahydrofuranylmethyl, Tetrahydrofuranylethyl, Dioxolanylmethyl, Dioxolanylethyl, Dioxanylmethyl oder Dioxanylethyl, für jeweils unsubstituiertes oder ein- bis fünffach, gleich oder verschieden durch Methyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl, Cyclohexylmethyl, Cyclohexylethyl oder Cyclohexylpropyl oder für jeweils unsubstituiertes oder ein- bis dreifach, gleich oder verschieden substituiertes Phenyl, Benzyl, Phenethyl, Phenylpropyl oder Phenylbutyl, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl.

Die Decalinamine der Formel (VII) sind teilweise bekannt (vergl. z.B. J. Amer. Chem. Soc. 91, 533-535 [1969]; US 3 927 020; Synthesis 1987, 1005-1007). Zum größten Teil sind die jedoch erfindungsgemäße Verbindungen und erhältlich mit Hilfe der erfindungsgemäßen Verfahren (a), (b) und (c).
Die zur Durchführung des erfindungsgemäßen Verfahrens (d) weiterhin als Ausgangsstoffe benötigten Alkylierungsmittel sind durch die Formel (VIII) allgemein definiert. In dieser Formel (VIII) steht
$R^{1-1}$ vorzugsweise für jeweils geradkettiges oder verzweigtes Alkyl mit 3 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen oder für jeweils geradkettiges oder verzweigtes Tetrahydrofuranylalkyl, Tetrahydropyranylalkyl, Dioxolanylalkyl oder Dioxanylalkyl mit jeweils 1 bis 4 Kohlenstoffatomen im Alkylteil oder für jeweils unsubstituiertes oder im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und 1 bis 4 Kohlenstoffatomen im geradkettigen oder verzweigten Alkylteil, wobei als Substituenten infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;
$R^{1-1}$ steht außerdem für jeweils unsubstituiertes oder einfach bis mehrfach, gleich oder verschieden im Arylteil substituiertes Arylalkyl oder Arylalkenyl mit jeweils 6 bis 10 Kohlenstoffatomen im Arylteil und bis zu 6 Kohlenstoffatomen im geradkettigen oder verzweigten Alkyl- bzw. Alkenylteil, wobei als Arylsubstituenten infrage kommen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen;
$R^{1-1}$ steht besonders bevorzugt für jeweils geradkettiges oder verzweigtes Propyl, Butyl, Pentyl, Hexyl, Heptyl, Octyl, Allyl, Butenyl, Pentenyl, Propargyl, Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Tetrahydrofuranylmethyl, Tetrahydropyranylmethyl, Dioxolanylmethyl, Dioxolanylethyl, Dioxanylmethyl oder Dioxanylethyl, für jeweils unsubstituiertes oder ein-bis fünffach, gleich oder verschieden durch Fluor, Chlor, Brom, Methyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl, Cyclohexylmethyl, Cyclohexylethyl oder Cyclohexylpropyl oder für jeweils unsubstituiertes oder ein- bis dreifach, gleich oder verschieden substituiertes Benzyl oder Phenylethyl, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl.

E steht für einen bei Alkylierungsmitteln üblichen Abgangsrest, wie beispielsweise Halogen, Alkylsulfonyloxy oder p-Toluolsulfonyloxy;
E steht insbesondere für Chlor, Brom oder Iod.

Die Alkylierungsmittel der Formel (VIII) sind bekannt oder erhältlich in Analogie zu bekannten Verfahren (vergl. z.B. J. med. Chem. 18, 996-1000 [1975]; Bull. Soc. Chim. France 1971. 1080-1087; J. org. Chem. 34, 212-218 [1969]; Bull. Soc. Chim. France 1972, 2445-2459; US 3 951 640; US 3 901 902).

Zur Durchführung der erfindungsgemäßen Verfahren (a) und (c) ist die Gegenwart eines geeigneten Reduktionsmittels erforderlich. Als solche kommen alle üblicherweise für derartige reduktive Aminierungsreaktionen einsetzbaren Reduktionsmittel infrage.
Vorzugsweise verwendet man molekularen Wasserstoff in Gegenwart eines geeigneten Hydrierkatalysators, wie beispielsweise Raney-Nickel, Platin oder Palladium oder auch komplexe Hydride, wie beispielsweise Lithiumaluminiumhydrid, Natriumborhydrid, Natriumcyanoborhydrid oder komplexe Aluminiumhydride, wie Diisopropylaluminiumhydrid oder Diisobutylaluminiumhydrid gegebenenfalls auch in Gegenwart von Natriumhydrid.
(Vergl. hierzu C.Ferri "Reaktionen der organischen Synthese" S.85f, 98f, 133; Thieme Verlag Stuttgart 1978 und dort zitierte Literatur sowie "Organikum" S.542f, VEB Deutscher Verlag der Wissenschaften, Berlin 1981).

Als Verdünnungsmittel zur Durchführung der erfindungsgemäßen Verfahren (a) und (c) kommen alle üblicherweise für derartige reduktive Aminierungsreaktionen einsetzbaren Verdünnungsmittel infrage. Mit besonderem Vorzug verwendet man halogenierte Kohlenwasserstoffe, wie Dichlormethan, Chloroform oder Tetrachlorkohlenstoff; Ether, wie Diethylether, Diisopropylether, Dioxan, Tetrahydrofuran oder Ethylenglykoldimethylether; Alkohole, wie Methanol, Ethanol, n- oder i-Propanol oder Ethylenglykolmonomethylether, gegebenenfalls auch in Mischung mit Wasser oder reines Wasser als Verdünnungsmittel.

Die erfindungsgemäßen Verfahren (a) und (c) können gegebenenfalls auch in einem Zweiphasensystem, wie beispielsweise Wasser/Chloroform oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-dimethyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid, Tris-[2-(2-methoxyethoxy)-ethyl]-amin oder Methyltridecylammoniumchlorid.

Die erfindungsgemäßen Verfahren (a) und (c) können in Abhängigkeit vom verwendeten Reduktionsmittel gegebenenfalls auch in Gegenwart eines geeigneten Reaktionshilfsmittels durchgeführt werden. Als solche kommen alle üblichen anorganischen oder organischen Basen oder Säuren infrage. Hierzu gehören als Basen beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat oder als Säuren anorganische Mineralsäuren, wie Salzsäure oder Schwefelsäure oder organische Säuren, wie beispielsweise Essigsäure.

Die Reaktionstemperaturen können bei der Durchführung der erfindungsgemäßen Verfahren (a) und (c) in einem größeren Bereich variiert werden. Im allgemeinen arbeitet man bei Temperaturen zwischen -20 °C und 250 °C, vorzugsweise bei Temperaturen zwischen 0 °C und 150 °C.

Die erfindungsgemäßen Verfahren (a) und (c) können gegebenenfalls auch unter erhöhtem Druck durchgeführt werden. Vorzugsweise arbeitet man in diesen Fällen in Druckbereichen zwischen 1 und 300 bar, insbesondere zwischen 20 und 150 bar.

Zur Durchführung des erfindungsgemäßen Verfahrens (a) setzt man pro Mol an Decalinon der Formel (II) im allgemeinen 1,0 bis 5,0 Mol, vorzugsweise 1,0 bis 3,0 Mol an Amin der Formel (III) und 1,0 bis 20,0 Mol, vorzugsweise 0,5 bis 5,0 Mol an Reduktionsmittel ein.

Die Reaktionsdurchführung, Aufarbeitung und Isolierung der Reaktionsprodukte erfolgt nach allgemein üblichen Methoden (vergl. auch die Herstellungsbeispiele).

Zur Durchführung des erfindungsgemäßen Verfahrens (c) setzt man pro Mol an Aldehyd oder Keton der Formel (VI) im allgemeinen 1,0 bis 5,0 Mol, vorzugsweise 1,0 bis 3,0 Mol an Decalinamin der Formel (V) und gegebenenfalls 0,2 bis 20,0 Mol, vorzugsweise 0,5 bis 5,0 Mol an Reduktionsmittel ein.

Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (b) kommen alle unter Hydrierungsbedingungen inerten Solventien in Betracht. Vorzugsweise verwendbar sind Kohlenwasserstoffe, wie Petrolether, Pentan, Hexan, Heptan, Cyclohexan; Alkohole, wie Methanol, Ethanol, n-Propanol und i-Propanol; oder Ether, wie Diethylether, Dioxan, Tetrahydrofuran oder Ethylenglykoldimethyl- oder -diethylether. Es kann jedoch auch ohne Verdünnungsmittel gearbeitet werden.

Als Hydrierkatalysatoren können bei dem erfindungsgemäßen Verfahren (b) alle üblicherweise für derartige Reaktionen verwendbaren Katalysatoren eingesetzt werden. Vorzugsweise verwendet man Raney-Nickel oder Edelmetallkatalysatoren, wie Palladium, Ruthenium, Palladiumoxid, Platin oder Platinoxid,

gegebenenfalls auf einem geeigneten Trägerstoff, wie z.B. Kohle oder Aluminiumoxid.

Die Reaktionstemperaturen können bei dem erfindungsgemäßen Verfahren (b) in einem größeren Bereich variiert werden. Im allgemeinen arbeitet man bei Temperaturen zwischen 50 °C und 250 °C, vorzugsweise bei Temperaturen zwischen 80 °C und 200 °C.

Das erfindungsgemäße Verfahren (b) kann bei Normaldruck, aber auch bei erhöhtem Druck durchgeführt werden. Bevorzugt wird unter erhöhtem Druck gearbeitet. Im allgemeinen arbeitet man in Druckbereichen zwischen 5 und 300 atm, vorzugsweise zwischen 10 und 200 atm.

Bei der Durchführung des erfindungsgemäßen Verfahrens (b) setzt man pro Mol an substituiertem ß-Naphthylamin der Formel (IV) im allgemeinen 5,0 bis 30 Mol Wasserstoff und 0,001 bis 10 Mol, vorzugsweise 0,01 bis 0,1 Mol Katalysator ein.

Bei der Durchführung des erfindungsgemäßen Verfahrens (b) geht man im allgemeinen so vor, daß man die Verbindungen der Formel (IV) in Gegenwart eines Verdünnungsmittels bei der jeweils gewünschten Temperatur mit Wasserstoff in Gegenwart des Hydrierungskatalysators im Autoklaven umsetzt. Die Aufarbeitung und Isolierung der Reaktionsprodukte erfolgt nach üblichen Methoden.

Als Verdünnungsmittel zur Durchführung des erfindungsgemäßen Verfahrens (d) kommen inerte organische Lösungsmittel oder wäßrige Systeme infrage.
Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl- oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid, Ester, wie Essigsäureethylester; oder Sulfoxide, wie Dimethylsulfoxid.
Das erfindungsgemäße Verfahren (d) kann gegebenenfalls auch in einem Zweiphasensystem, wie beispielsweise Wasser/Toluol oder Wasser/Dichlormethan, gegebenenfalls in Gegenwart eines Phasentransferkatalysators durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyldimethylammoniummethylsulfat, Dimethyl-$C_{12}$/$C_{14}$-alkyl-benzylammoniumchlorid, Tetrabutylammoniumhydroxid, 15-Krone-5, 18 Krone-6, Triethylbenzylammoniumchlorid, Trimethylbenzylammoniumchlorid. Es ist auch möglich, das erfindungsgemäße Verfahren (d) ohne Zusatz eines Lösungsmittels durchzuführen.

Als Reaktionshilfsmittel zur Durchführung des erfindungsgemäßen Verfahrens (d) kommen alle üblicherweise verwendbaren anorganischen und organischen Basen infrage. Vorzugsweise verwendet man Alkalimetallhydroxide, -carbonate oder -hydrogencarbonate, wie beispielsweise Natriumhydroxid, Natriumcarbonat oder Natriumhydrogencarbonat oder auch tertiäre Amine, wie beispielweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU). Es ist auch möglich, die als Reaktionsteilnehmer verwendeten Decalinamine der Formel (VII) in entsprechendem Überschuß gleichzeitig als Reaktionshilfsmittel einzusetzen.

Die Reaktionstemperaturen können bei der Durchführung des erfindungsgemäßen Verfahrens (d) in einem größeren Bereich variiert werden. Im allgemeinen arbeitet man bei Temperaturen zwischen - 20 °C und + 180 °C, vorzugsweise bei Temperaturen zwischen 20 °C und +150 °C.

Zur Durchführung des erfindungsgemäßen Verfahrens (d) setzt man pro Mol an Decalinamin der Formel (VII) im allgemeinen 1,0 bis 3,0 Mol, vorzugsweise 1,0 bis 1,5 Mol an Alkylierungsmittel der Formel (VIIIa) und gegebenenfalls 1,0 bis 3,0 Mol, vorzugsweise 1,0 bis 1,5 Mol an Reaktionshilfsmittel sowie gegebenenfalls 0,1 bis 1,0 Mol an Phasentransferkatalysator ein.

Die Reaktionsdurchführung, Aufarbeitung und Isolierung der Reaktionsprodukte der Formel (Ib) erfolgt in beiden Fällen nach üblichen Methoden.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide, geeignet.
Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.
Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die neuen Wirkstoffe können mit besonders gutem Erfolg zur Bekämpfung von Getreidekrankheiten, wie beispielsweise gegen den Erreger des echten Getreidemehltaus (Erysiphe graminis) oder zur Bekämpfung von Reiskrankheiten, wie beispielsweise gegen den Erreger der Reisfleckenkrankheit (Pyricularia oryzae) oder zur Bekämpfung von Krankheiten im Obst- und Gemüseanbau, wie beispielsweise gegen den Erreger des echten Gurkenmehltaus (Sphaerotheca fuliginea) eingesetzt werden.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele:

Beispiel 1:

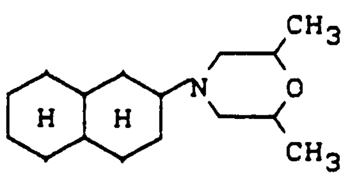

(Verfahren a)

Eine Mischung von 12,5 g (0,08 Mol) Decalin-2-on, 15 g (0,13 Mol) cis-2,6-Dimethylmorpholin und 3 g Palladium (einprozentig auf Aluminiumoxid) wird bei 100 °C und 50 bar 5 Stunden lang mit Wasserstoff hydriert. Zur Aufarbeitung wird der Katalysator abfiltriert und das Filtrat im Vakuum eingeengt.

Man erhält 17,9 g (89 % der Theorie) an cis-2,6-Dimethyl-4-(2-decalinyl)-morpholin vom Brechungsindex $n_D^{20}$ 1,4790, welches als Gemisch aus cis/trans-Isomeren des Decalins vorliegt.

Beispiel 2:

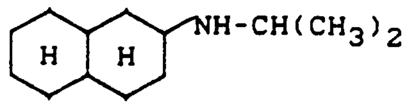

(Verfahren b)

Eine Mischung aus 150 g (0,81 Mol) 2-Isopropylaminonaphthalin und 30 g Ruthenium auf Aktivkohle (5 prozentig) in 750 ml Tetrahydrofuran wird bei 150 °C und 150 bar 3 Stunden lang mit Wasserstoff hydriert. Zur Aufarbeitung wird der Katalysator abfiltriert und das Filtrat im Vakuum eingeengt.

Man erhält 107 g (67 % der Theorie) an N-Isopropyl-2-decalinamin vom Brechungsindex $n_D^{20}$ 1,4820.

Beispiel 3:

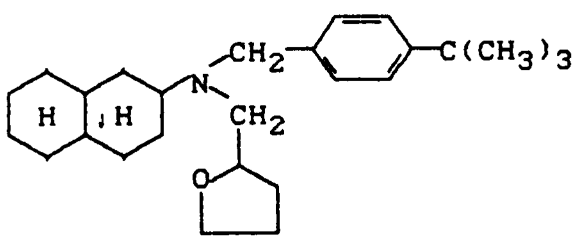

(Verfahren d

Eine Mischung aus 5 g (0,021 Mol) 2-[N-(2-Tetrahydrofuranylmethyl)-amino]-decalin, 6,81 g (0,03 Mol) 4-t-Butylbenzylbromid und 7 g (0,06 Mol) Kaliumcarbonat in 20 ml Acetonitril wird 16 Stunden bei Rückflußtemperatur gerührt, anschließend filtriert, im Vakuum eingeengt, der Rückstand in Dichlormethan aufgenommen, mit Wasser gewaschen, über Natriumsulfat getrocknet und im Vakuum eingeengt.

Man erhält 7,3 g (91 % der Theorie) an 2-[N-(4-t-Butylbenzyl)-N-(2-tetrahydrofuranylmethyl)-amino]-decalin ($^1$H-NMR-Spektrum: Multiplett bei 3,5- 4,1 ppm (4H) und 3,0-3,5 ppm (2H)).

Beispiel 4:

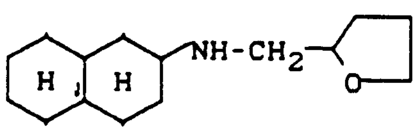

(Verfahren a)

Eine Mischung von 10 g (0,066 Mol) Decalin-2-on, 20 g (0,2 Mol) Tetrahydrofuranylmethylamin und 3 g Palladium (einprozentig auf Aluminiumoxid) wird bei 100 °C und 50 bar 5 Stunden lang mit Wasserstoff hydriert. Zur Aufarbeitung wird der Katalysator abfiltriert und das Filtrat im Vakuum eingeengt.

Man erhält 15 g (96 % der Theorie) an 2-[N-(2-Tetrahydrofuranylmethyl)-amino]-decalin vom Brechungsindex $n_D^{20}$ 1,4998.

In entsprechender Weise und gemäß den allgemeinen Angaben zur Herstellung erhält man die folgenden substituierten Decalinamine der allgemeinen Formel

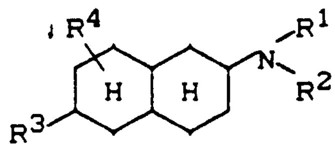 (I)

| Bsp. Nr. | 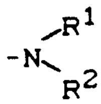 | $R^3$ | $R^4$ | physikalische Eigenschaften |
|---|---|---|---|---|
| 5 | 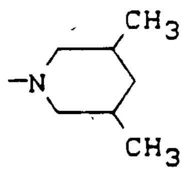 (cis) | H | H | $n_D^{20}$ 1,4964 |
| 6 | 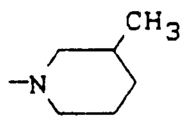 | H | H | $n_D^{20}$ 1,5016 |
| 7 | 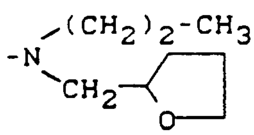 | H | H | $n_D^{20}$ 1,4953 |
| 8 | $-NH-C(CH_3)_3$ | H | H | Fp: 98-105° C |
| 9 | 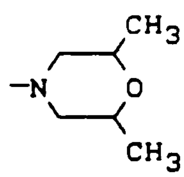 (cis) | H | H | $n_D^{25}$ 1,4915 (trans-Decalinyl) |
| 10 | 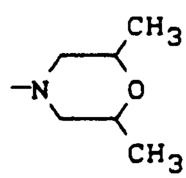 (cis) | H | H | $n_D^{25}$ 1,4876 (cis-Decalinyl) |

| Bsp. Nr. | 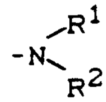 | $R^3$ | $R^4$ | physikalische Eigenschaften |
|---|---|---|---|---|
| 11 | 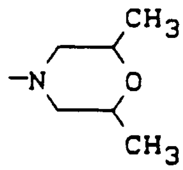 (cis) | H | H | $n_D^{20}$ 1,4924 (Isomeres 1) |
| 12 | 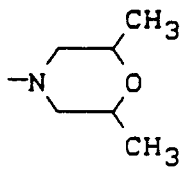 (cis) | $CH_3$ | H | $n_D^{20}$ 1,4952 (Isomerengemisch) |
| 13 | 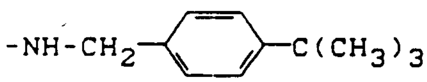 | H | H | $n^{25}$ 1,5227 (Isomeres 2) |
| 14 | 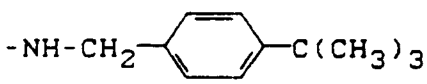 | H | H | $n_D^{25}$ 1,5211 (Isomeres 1) |
| 15 | 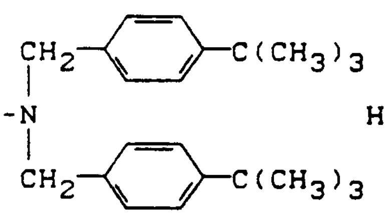 | H | H | $n_D^{25}$ 1,5355 |
| 16 | 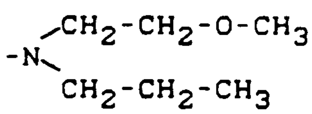 | H | H | $n_D^{25}$ 1,4794 |
| 17 | 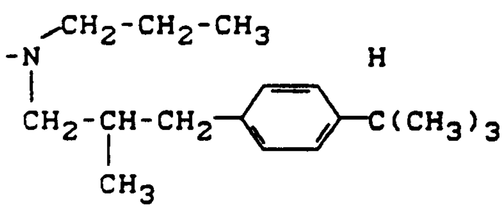 | H | H | $n_D^{25}$ 1,5090 |

| Bsp. Nr. | 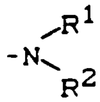 | $R^3$ | $R^4$ | physikalische Eigenschaften |
|---|---|---|---|---|
| 18 | $-NH-CH_2CH_2-O-CH_3$ | H | H | $n_D^{20}$ 1,4836 |
| 19 | 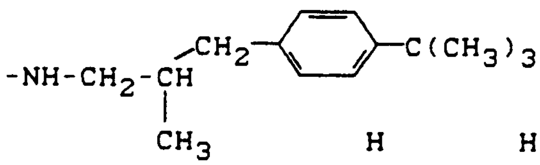 | H | H | $n_D^{25}$ 1,5132 |

Anwendungsbeispiele:

Im dem folgenden Anwendungsbeispiel wurde die nachstehend aufgeführte Verbindung als Vergleichssubstanz eingesetzt:

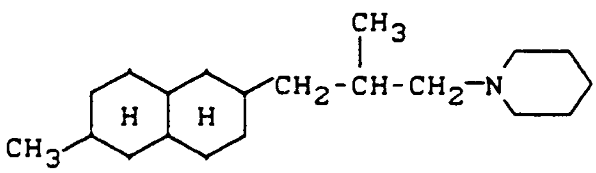

(A)

2-[2-Methyl-3-(piperidin-1-yl)-propyl]-6-methyldecalin (bekannt aus EP 254 150)

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Gegenüber der Verbindung (A) zeigen die Verbindungen der Herstellungsbeispiele (1), (5) und (10) bei einer beispielhaften Konzentration von 0,0025 Gew.-% einen um 35 % höheren Wirkungsgrad.

**Patentansprüche**

1. Substituierte Decalinamine der allgemeinen Formel (I),

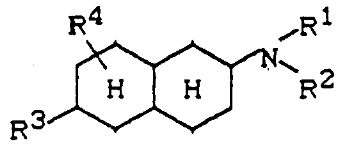

(I)

in welcher

$R^1$ für Alkyl mit mehr als zwei Kohlenstoffatomen, für Alkenyl, Alkinyl, Alkoxyalkyl, Dialkoxyalkyl, Hydroxyalkyl, Hydroxyalkoxyalkyl, Tetrahydrofuranylalkyl, Tetrahydropyranylalkyl, Dioxolanylalkyl, Dioxanylalkyl oder für jeweils unsubstituiertes oder substituiertes Cycloalkylalkyl, Cycloalkyl, Aralkyl, Aralkenyl oder Aryl steht,

$R^2$ für Wasserstoff, Alkyl, Alkenyl, Alkinyl, Alkoxyalkyl, Dialkoxyalkyl, Hydroxyalkyl, Hydroxyalkoxyalkyl, Tetrahydrofuranylalkyl, Tetrahydropyranylalkyl, Dioxolanylalkyl, Dioxanylalkyl oder für jeweils unsubstituiertes oder substituiertes Cycloalkylalkyl, Cycloalkyl, Aralkyl, Aralkenyl oder Aryl steht oder

$R^1$ und $R^2$ gemeinsam mit dem Stickstoffatom, an welches sie gebunden, sind für einen unsubstituierten oder substituierten gesättigten Heterocyclus stehen, dem gegebenenfalls weitere Heteroatome enthalten kann und

$R^3$ und $R^4$ unabhängig voneinander jeweils für Wasserstoff, Alkyl, Halogenalkyl, Alkoxy oder Halogenalkoxy stehen,

wobei jedoch $R^1$ und $R^2$ gemeinsam mit dem Stickstoffatom, an welches sie gebunden sind, nur dann für einen unsubstituierten oder substituierten Pyrrolidinylrest stehen, wenn $R^3$ und $R^4$ nicht gleichzeitig für Wasserstoff stehen, und für den Fall, daß $R^4$ für 9-Methyl steht, $R^3$ nicht für Wasserstoff steht,

sowie deren Säureadditionssalze.

2. Substituierte Decalinamine gemäß Anspruch 1 wobei in der Formel (I)

$R^1$ für jeweils geradkettiges oder verzweigtes Alkyl mit 3 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 8 Kohlenstoffatomen oder Hydroxyalkoxyalkyl mit 2 bis 6 Kohlenstoffatomen in den einzelnen Alkylteilen, für jeweils geradkettiges oder verzweigtes Tetrahydrofuranylalkyl, Tetrahydropyranylalkyl, Dioxolanylalkyl oder Dioxanylalkyl mit jeweils 1 bis 4 Kohlenstoffatomen im Alkylteil oder für jeweils unsubstituiertes oder im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und 1 bis 4 Kohlenstoffatomen im geradkettigen oder verzweigten Alkylteil steht, wobei als Substituenten jeweils infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;
oder für jeweils unsubstituiertes oder einfach bis mehrfach, gleich oder verschieden im Arylteil substituiertes Arylalkyl, Arylalkenyl oder Aryl mit jeweils 6 bis 10 Kohlenstoffatomen im Arylteil und bis zu 6 Kohlenstoffatomen im geradkettigen oder verzweigten Alkyl- bzw. Alkenylteil steht, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen,

$R^2$ für Wasserstoff, für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 8 Kohlenstoffatomen oder Hydroxyalkoxyalkyl mit 2 bis 6 Kohlenstoffatomen in den einzelnen Alkylteilen, für jeweils geradkettiges oder verzweigtes Tetrahydrofuranylalkyl, Tetrahydropyranylalkyl, Dioxolanylalkyl oder Dioxanylalkyl mit jeweils 1 bis 4 Kohlenstoffatomen im Alkylteil oder für jeweils unsubstituiertes oder im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und 1 bis 4 Kohlenstoffatomen im geradkettigen oder verzweigten Alkylteil steht, wobei als Substituenten jeweils infrage kommen: jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;

oder für jeweils unsubstituiertes oder einfach bis mehrfach, gleich oder verschieden im Arylteil substituiertes Arylalkyl, Arylalkenyl oder Aryl mit jeweils 6 bis 10 Kohlenstoffatomen im Arylteil und gegebenenfalls bis zu 6 Kohlenstoffatomen im geradkettigen oder verzweigten Alkyl- bzw. Alkenylteil steht, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^1$ und $R^2$ gemeinsam mit dem Stickstoffatom, an welches sie gebunden sind, für einen unsubstituierten oder einfach bis mehrfach, gleich oder verschieden substituierten, gesättigten 5- bis 7-gliedrigen Heterocyclus stehen, der gegebenenfalls ein weiteres Heteroatom, enthalten kann, wobei als Substituenten infrage kommen: jeweils geradkettiges oder verzweigtes Alkyl oder Hydroxyalkyl mit jeweils 1 bis 4 Kohlenstoffatomen und

$R^3$ und $R^4$ unabhängig voneinander jeweils für Wasserstoff, für jeweils geradkettiges oder verzweigtes Alkyl oder Alkoxy mit jeweils 1 bis 4 Kohlenstoffatomen oder für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen stehen.

**3.** Substituierte Decalinamine gemäß Anspruch 1, wobei in der Formel (I)

$R^1$ für jeweils geradkettiges oder verzweigtes Propyl, Butyl, Pentyl, Hexyl, Heptyl, Octyl, Allyl, Butenyl, Pentenyl, Propargyl, Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, für Tetrahydrofuranylmethyl, Tetrahydropyranylmethyl, Dioxolanylmethyl, Dioxolanylethyl, Dioxanylmethyl oder Dioxanylethyl, für jeweils unsubstituiertes oder ein- bis fünffach, gleich oder verschieden durch Methyl, Trifluormethyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl, Cyclohexylmethyl, Cyclohexylethyl oder Cyclohexylpropyl oder für jeweils unsubstituiertes oder ein- bis dreifach, gleich oder verschieden substituiertes Phenyl, Benzyl oder Phenethyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl und

$R^2$ für Wasserstoff, Methyl, Ethyl, für jeweils geradkettiges oder verzweigtes Propyl, Butyl, Pentyl, Hexyl, Heptyl, Octyl, Allyl, Butenyl, Pentenyl, Propargyl, Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, für Tetrahydrofuranylmethyl, Tetrahydropyranylmethyl, Dioxolanylmethyl, Dioxolanylethyl, Dioxanylmethyl oder Dioxanylethyl, für jeweils unsubstituiertes oder ein- bis fünffach, gleich oder verschieden durch Methyl, Trifluormethyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils unsubstituiertes oder ein- bis dreifach, gleich oder verschieden substituiertes Phenyl, Benzyl, Phenylethyl, Phenylpropyl oder Phenylbutyl steht, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl oder

$R^1$ und $R^2$ gemeinsam mit dem Stickstoffatom, an welches sie gebunden sind, für einen unsubstituierten oder ein- bis dreifach, gleich oder verschieden substituierten Heterocyclus der Formel

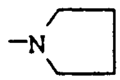 ; 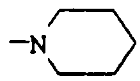 ; 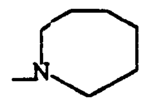 ; 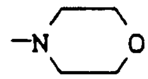

oder 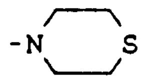

stehen, wobei als Substituenten jeweils infrage kommen: Methyl, Ethyl oder Hydroxymethyl und

$R^3$ und $R^4$ unabhängig voneinander jeweils für Wasserstoff, Methyl, Ethyl, n- oder i-Propyl, n-, i-, s- oder t-Butyl, für Trifluormethyl, Difluormethyl, Fluormethyl, Chlordifluormethyl, Dichlorfluormethyl, für Methoxy, Ethoxy, n- oder i-Propoxy, Trifluormethoxy oder Difluormethoxy stehen.

**4.** Substituierte Decalinamine gemäß Anspruch 1, wobei in der Formel (I)

$R^1$ für jeweils geradkettiges oder verzweigtes Propyl, Butyl, Pentyl, Hexyl, Allyl, Butenyl, Pentenyl, Propargyl, Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Tetrahydrofuranylmethyl, Tetrahydropyranylmethyl, Dioxolanylmethyl, Dioxolanylethyl, Dioxanylmethyl, Cyclopropylmethyl, Dichlorcyclopropylmethyl, Dimethylcyclopropylmethyl, Dichlordimethylcyclopropylmethyl, Cyclopentyl, Cyclohexyl oder Cyclohexylmethyl steht und

$R^2$ für Wasserstoff, Methyl, Ethyl oder für jeweils gegebenenfalls geradkettiges oder verzweigtes Propyl, Butyl, Pentyl, Hexyl, Allyl, Butenyl, Pentenyl, Propargyl, Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Tetrahydrofuranylmethyl, Tetrahydropyranylmethyl, Dioxolanylmethyl, Dioxolanylethyl, Dioxanylmethyl, Dioxanylethyl, Cyclopropylmethyl, Dimethylcyclopropylmethyl, Cyclopentyl, für jeweils unsubstituiertes oder einfach bis dreifach, gleich oder verschieden durch Methyl oder t-Butyl substituiertes Cyclohexyl, Cyclohexylmethyl, Benzyl, Phenylethyl oder Phenylpropyl steht oder

$R^1$ und $R^2$ gemeinsam mit dem Stickstoffatom, an welches sie gebunden sind, für einen unsubstituierten oder ein- bis dreifach, gleich oder verschieden substituierten Heterocyclus der Formel

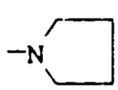 ; 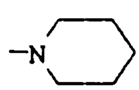 ; 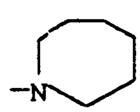 oder 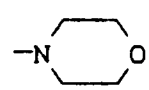

stehen

wobei als Substituenten jeweils infrage kommen: Methyl, Ethyl oder Hydroxymethyl und

$R^3$ und $R^4$ unabhängig voneinander jeweils für Wasserstoff, Methyl, Ethyl, n- oder i-Propyl, t-Butyl oder Trifluormethyl stehen.

**5.** Verfahren zur Herstellung substituierter Decalinamine der allgemeinen Formel (I),

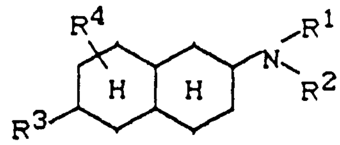

(I)

in welcher

$R^1$ für Alkyl mit mehr als zwei Kohlenstoffatomen, für Alkenyl, Alkinyl, Alkoxyalkyl, Dialkoxyalkyl, Hydroxyalkyl, Hydroxyalkoxyalkyl, Tetrahydrofuranylalkyl, Tetrahydropyranylalkyl, Dioxolanylalkyl, Dioxanylalkyl oder für jeweils unsubstituiertes oder substituiertes Cycloalkylalkyl, Cycloalkyl, Aralkyl, Aralkenyl oder Aryl steht,

$R^2$ für Wasserstoff, Alkyl, Alkenyl, Alkinyl, Alkoxyalkyl, Dialkoxyalkyl, Hydroxyalkyl, Hydroxyalkoxyalkyl, Tetrahydrofuranylalkyl, Tetrahydropyranylalkyl, Dioxolanylalkyl, Dioxanylalkyl oder für jeweils unsubstituiertes oder substituiertes Cycloalkylalkyl, Cycloalkyl, Aralkyl, Aralkenyl oder Aryl steht oder

$R^1$ und $R^2$ gemeinsam mit dem Stickstoffatom, an welches sie gebunden, sind für einen unsubstituierten oder substituierten gesättigten Heterocyclus stehen, der gegebenenfalls weitere Heteroatome enthalten kann und

$R^3$ und $R^4$ unabhängig voneinander jeweils für Wasserstoff, Alkyl, Halogenalkyl Alkoxy oder Halogenalkoxy stehen,

wobei jedoch $R^1$ und $R^2$ gemeinsam mit dem Stickstoffatom, an welches sie gebunden sind, nur dann für einen unsubstituierten oder substituierten Pyrrolidinylrest stehen, wenn $R^3$ und $R^4$ nicht gleichzeitig für Wasserstoff stehen, und für den Fall, daß $R^4$ für 9-Methyl steht, $R^3$ nicht für Wasserstoff steht, sowie deren Säureadditionssalze dadurch gekennzeichnet, daß man

(a) Decalinone der Formel (II),

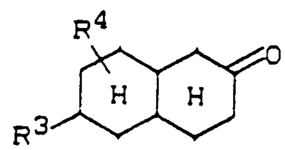

(II)

in welcher

$R^3$ und $R^4$ die oben angegebene Bedeutung haben,

mit Aminen der Formel (III),

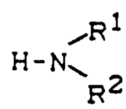

(III)

in welcher

$R^1$ und $R^2$ die oben angegebene Bedeutung haben,

in Gegenwart eines Reduktionsmittels, gegebenenfalls in Gegenwart eines Verdünnungsmittels und gegebenenfalls in Gegenwart eines Reaktionshilfsmittels umsetzt oder daß man

(b) substituierte $\beta$-Naphthylamine der Formel (IV),

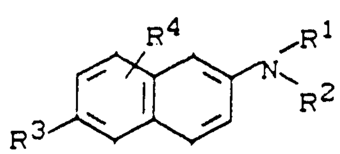

(IV)

in welcher
$R^1$, $R^2$, $R^3$ und $R^4$ die oben angegebene Bedeutung haben,
mit Wasserstoff in Gegenwart eines Katalysators und gegebenenfalls in Gegenwart eines Verdünnungsmittels hydriert;
und gegebenenfalls anschließend eine Säure addiert.

**6.** Verfahren zur Herstellung substituierter Decalinamine der Formel (Ia),

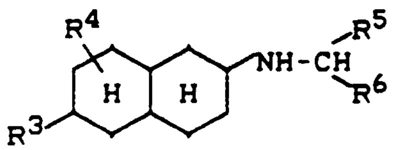

(Ia)

in welcher

$R^5$ für Alkyl, Alkenyl, Alkinyl, Alkoxy, Alkoxyalkyl, Dialkoxyalkyl, Hydroxyalkyl, Hydroxyalkoxyalkyl, Tetrahydrofuranyl, Tetrahydropyranyl, Dioxolanyl, Dioxanyl, für Tetrahydrofuranylalkyl, Tetrahydropyranylalkyl, Dioxolanylalkyl, Dioxanylalkyl oder für jeweils unsubstituiertes oder substituiertes Cycloalkylalkyl, Cycloalkyl, Aralkyl, Aralkenyl oder Aryl steht,
$R^6$ für Wasserstoff oder Alkyl steht und
$R^3$ und $R^4$ die in Anspruch 5 angegebene Bedeutung haben,

wobei $R^5$ nur dann für Methyl steht, wenn $R^6$ nicht gleichzeitig für Wasserstoff steht,
dadurch gekennzeichnet, daß man Decalinamine der Formel (V),

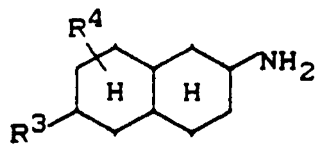

(V)

in welcher
$R^3$ und $R^4$ die in Anspruch 5 angegebene Bedeutung haben,
mit Aldehyden oder Ketonen der Formel (VI),

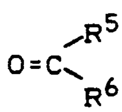

(VI)

in welcher
$R^5$ und $R^6$ die oben angegebene Bedeutung haben,
in Gegenwart eines Reduktionsmittels, gegebenenfalls in Gegenwart eines Verdünnungsmittels und gegebenenfalls in Gegenwart eines Reaktionshilfsmittels umsetzt und gegebenenfalls anschließend eine Säure addiert.

**7.** Verfahren zur Herstellung substituierter Decalinamine der Formel (Ib),

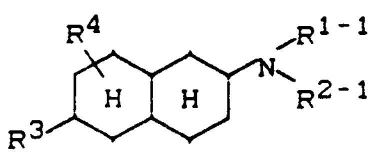

(Ib)

in welcher

$R^{1-1}$ für Alkyl mit mehr als zwei Kohlenstoffatomen, für Alkenyl, Alkinyl, Alkoxyalkyl, Dialkoxyalkyl, Hydroxyalkyl, Hydroxyalkoxyalkyl, Tetrahydrofuranylalkyl, Tetrahydropyranylalkyl, Dioxolanylalkyl, Dioxanylalkyl, für jeweils unsubstituiertes oder substituiertes Cycloalkylalkyl, Cycloalkyl, Aralkyl oder Aralkenyl steht,

$R^{2-1}$ für Wasserstoff, Alkyl, Alkenyl, Alkinyl, Alkoxyalkyl, Dialkoxyalkyl, Hydroxyalkyl, Hydroxyalkoxyalkyl, Tetrahydrofuranylalkyl, Tetrahydropyranylalkyl, Dioxolanylalkyl, Dioxanylalkyl oder für jeweils unsubstituiertes oder substituiertes Cycloalkyl, Aralkyl, Aralkenyl oder Aryl steht und

$R^3$ und $R^4$ die in Anspruch 5 angegebene Bedeutung haben,

dadurch gekennzeichnet, daß man Decalinamine der Formel (VII),

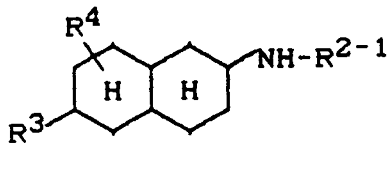

(VII)

in welcher

$R^{2-1}$, $R^3$ und $R^4$ die oben angegebene Bedeutung haben,

mit Alkylierungsmitteln der Formel (VIII),

$R^{1-1}$ - E (VIII)

in welcher

$R^{1-1}$ die oben angegebene Bedeutung hat und

E für eine elektronenanziehende Abgangsgruppe steht,

gegebenenfalls in Gegenwart eines Verdünnungsmittels und gegebenenfalls in Gegenwart eines Reaktionshilfsmittels umsetzt und gegebenenfalls anschließend eine Säure addiert.

**8.** Schädlingsbekämpfungsmittel, gekennzeichnet durch einen Gehalt an mindestens einem Decalinamin der Formel (I) nach den Ansprüche 1 bis 7.

**9.** Verwendung von Decalinaminen der Formel (I) nach den Ansprüchen 1 bis 7 zur Bekämpfung von Schädlingen.

**10.** Verfahren zur Bekämpfung von Schädlingen, dadurch gekennzeichnet, daß man Decalinamine der Formel (I) nach den Ansprüchen 1 bis 7 auf Schädlinge und/oder ihren Lebensraum einwirken läßt.

**11.** Verfahren zur Herstellung von Schädlingsbekämpfungsmitteln, dadurch gekennzeichnet, daß man Decalinamine der Formel (I) nach den Ansprüchen 1 bis 7 mit Streckmitteln und/oder oberflächenaktiven Mitteln vermischt.

## Claims

**1.** Substituted decalinamines of the general formula (I)

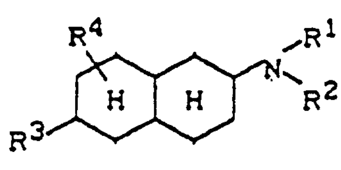

(I)

in which

$R^1$ represents alkyl having more than two carbon atoms, alkenyl, alkinyl, alkoxyalkyl, dialkoxyalkyl, hydroxyalkyl, hydroxyalkoxyalkyl, tetrahydrofuranylalkyl, tetrahydropyranylalkyl, dioxolanylalkyl, dioxanylalkyl or represents in each case unsubstituted or substituted cycloalkylalkyl, cycloalkyl, aralkyl, aralkenyl or aryl,

$R^2$ represents hydrogen, alkyl, alkenyl, alkinyl, alkoxyalkyl, dialkoxyalkyl, hydroxyalkyl, hydroxyalkoxyalkyl, tetrahydrofuranylalkyl, tetrahydropyranylalkyl, dioxolanylalkyl, dioxanylalkyl or represents in each case unsubstituted or substituted cycloalkylalkyl, cycloalkyl, aralkyl, aralkenyl or aryl, or

$R^1$ and $R^2$ , together with the nitrogen atom to which they are attached, represent an unsubstituted or substituted saturated heterocycle which may optionally contain further heteroatoms, and

$R^3$ and $R^4$ independently of one another each represent hydrogen, alkyl, halogenoalkyl, alkoxy or halogenoalkoxy,

$R^1$ and $R^2$ however, together with the nitrogen atom to which they are attached, only representing an unsubstituted or substituted pyrrolidinyl radical if $R^3$ and $R^4$ do not simultaneously represent hydrogen, and, in the case where $R^4$ represents 9-methyl, $R^3$ does not represent hydrogen,

and acid addition salts thereof.

**2.** Substituted decalinamines according to Claim 1, where in the formula (I)

$R^1$ represents in each case straight-chain or branched alkyl having 3 to 12 carbon atoms, alkenyl having 3 to 8 carbon atoms, alkinyl having 3 to 8 carbon atoms, hydroxyalkyl having 2 to 6 carbon atoms, alkoxyalkyl or dialkoxyalkyl in each case having 1 to 8 carbon atoms or hydroxyalkoxyalkyl having 2 to 6 carbon atoms in the individual alkyl moieties, or represents in each case straight-chain or branched tetrahydrofuranylalkyl, tetrahydropyranylalkyl, dioxolanylalkyl or dioxanylalkyl having in each case 1 to 4 carbon atoms in the alkyl moiety, or represents cycloalkyl or cycloalkylalkyl having in each case 3 to 7 carbon atoms in the cycloalkyl moiety and 1 to 4 carbon atoms in the straight-chain or branched alkyl moiety, each of which is unsubstituted or substituted in the cycloalkyl moiety once or more than once by identical or different substituents, possible substituents in each case being: halogen, in each case straight-chain or branched alkyl, alkoxy, halogenoalkyl or halogenoalkoxy having in each case 1 to 4 carbon atoms and optionally 1 to 9 identical or different halogen atoms;

or represents arylalkyl, arylalkenyl or aryl having in each case 6 to 10 carbon atoms in the aryl moiety and up to 6 carbon atoms in the straight-chain or branched alkyl or alkenyl moiety, which are in each case unsubstituted or substituted once or more than once in the aryl moiety by identical or different substituents, possible substituents of aryl being: halogen, cyano, nitro, in each case straight-chain or branched alkyl, alkoxy, alkylthio, halogenoalkyl, halogenoalkoxy, halogenoalkylthio, alkoxycarbonyl or alkoximinoalkyl having in each case 1 to 4 carbon atoms in the individual alkyl moieties and optionally 1 to 9 identical or different halogen atoms,

$R^2$ represents hydrogen, or represents in each case straight-chain or branched alkyl having 1 to 12 carbon atoms, alkenyl having 3 to 8 carbon atoms, alkinyl having 3 to 8 carbon atoms, hydroxyalkyl having 2 to 6 carbon atoms, alkoxyalkyl or dialkoxyalkyl having in each case 1 to 8 carbon atoms or hydroxyalkoxyalkyl having 2 to 6 carbon atoms in the individual alkyl moieties, or represents in each case straight-chain or branched tetrahydrofuranylalkyl, tetrahydropyranylalkyl, dioxolanylalkyl or dioxanylalkyl having in each case 1 to 4 carbon atoms in the alkyl moiety, or represents cycloalkyl or cycloalkylalkyl having in each case 3 to 7 carbon atoms in the cycloalkyl moiety and 1 to 4 carbon atoms in the straight-chain or branched alkyl moiety which are in each case unsubstituted or substituted once or more than once in the cycloalkyl moiety by identical or different substituents, possible substituents in each case being: in each case straight-chain or branched alkyl, alkoxy, halogenoalkyl or halogenoalkoxy having in each case 1 to 4 carbon atoms and optionally 1 to 9 identical or different halogen atoms;

or represents arylalkyl, arylalkenyl or aryl having in each case 6 to 10 carbon atoms in the aryl moiety and optionally up to 6 carbon atoms in the straight-chain or branched alkyl or alkenyl moiety which are in each case unsubstituted or substituted once or more than once in the aryl moiety by identical or different substituents, possible substituents of aryl in each case being: halogen, cyano, nitro, in each case straight-chain or branched alkyl, alkoxy, alkylthio, halogenoalkyl, halogenoalkoxy, halogenoalkylthio, alkoxycarbonyl or alkoximinoalkyl having in each case 1 to 4 carbon atoms in the individual alkyl moieties and optionally 1 to 9 identical or different halogen atoms, or

$R^1$ and $R^2$ , together with the nitrogen atom to which they are attached, represent a saturated 5- to 7-membered heterocycle which may optionally contain a further heteroatom and is unsubstituted or substituted once or more than once by identical or different substituents, possible substituents being: in each case straight-chain or branched alkyl or hydroxyalkyl having in each case 1 to 4 carbon atoms, and

$R^3$ and $R^4$ independently of one another in each case represent hydrogen, in each case straight-chain or branched alkyl or alkoxy having in each case 1 to 4 carbon atoms, or in each case straight-chain or branched halogenoalkyl or halogenoalkoxy having in each case 1 to 4 carbon atoms and 1 to 9 identical or different halogen atoms.

**3.** Substituted decalinamines according to Claim 1, where in the formula (I)

$R^1$ represents in each case straight-chain or branched propyl, butyl, pentyl, hexyl, heptyl, octyl, allyl, butenyl, pentenyl, propargyl, butinyl, hydroxyethyl, hydroxypropyl, methoxyethyl, ethoxyethyl, propoxyethyl, butoxyethyl, methoxypropyl, ethoxypropyl, propoxypropyl, butoxypropyl, hydroxyethoxyethyl, dimethoxyethyl, dimethoxypropyl, diethoxyethyl, tetrahydrofuranylmethyl, tetrahydropyranylmethyl, dioxolanylmethyl, dioxolanylethyl, dioxanylmethyl or dioxanylethyl, or represents cyclopropyl, cyclopropylmethyl, cyclopropylethyl, cyclopropylpropyl, cyclopentyl, cyclopentylmethyl, cyclohexyl, cyclohexylmethyl, cyclohexylethyl or cyclohexylpropyl, each of which is unsubstituted or substituted once to five times by identical or different substituents comprising methyl, trifluoromethyl, ethyl, n- or i-propyl, n-, i-, s- and/or t-butyl, or represents phenyl, benzyl or phenethyl, each of which is unsubstituted or substituted once to three times by identical or different substituents, possible substituents in each case being: fluoro, chloro, bromo, cyano, nitro, methyl, ethyl, n- or i-propyl, n-, i-, s- or t-butyl, methoxy, ethoxy, methylthio, trifluoromethyl, trifluoromethoxy, trifluoromethylthio, methoxycarbonyl, ethoxycarbonyl or methoximinomethyl, and

$R^2$ represents hydrogen, methyl, ethyl, in each case straight-chain or branched propyl, butyl, pentyl, hexyl, heptyl, octyl, allyl, butenyl, pentenyl, propargyl, butinyl, hydroxyethyl, hydroxypropyl, methoxyethyl, ethoxyethyl, propoxyethyl, butoxyethyl, methoxypropyl, ethoxypropyl, propoxypropyl, butoxypropyl, hydroxyethoxyethyl, dimethoxyethyl, dimethoxypropyl, diethoxyethyl, tetrahydrofuranylmethyl, tetrahydropyranylmethyl, dioxolanylmethyl, dioxolanylethyl, dioxanylmethyl or dioxanylethyl, or represents cyclopropyl, cyclopropylmethyl, cyclopropylethyl, cyclopropylpropyl, cyclopentyl, cyclopentylmethyl, cyclohexyl or cyclohexylmethyl which are in each case unsubstituted or substituted once to five times by identical or different substituents comprising methyl, trifluoromethyl, ethyl, n- or i-propyl, n-, i-, s- and/or t-butyl, or represents phenyl, benzyl, phenylethyl, phenylpropyl or phenylbutyl which are in each case unsubstituted or substituted once to three times by identical or different substituents, possible substituents in each case being:
fluorine, chlorine, bromine, cyano, nitro, methyl, ethyl, n- or i-propyl, n-, i-, s- or t-butyl, methoxy, ethoxy, methylthio, trifluoromethyl, trifluoromethoxy, trifluoromethylthio, methoxycarbonyl, ethoxycarbonyl or methoximinomethyl, or

$R^1$ and $R^2$ , together with the nitrogen atom to which they are attached, represent a heterocycle of the formula

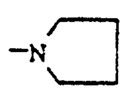 ; 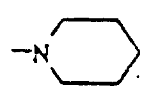 ; 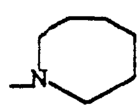 ; 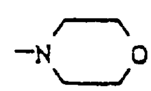

or 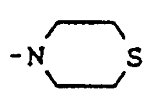

which is unsubstituted or substituted once to three times by identical or different

substituents, possible substituents in each case being: methyl, ethyl or hydroxymethyl, and

$R^3$ and $R^4$ independently of one another in each case represent hydrogen, methyl, ethyl, n- or i-propyl, n-, i-, s- or t-butyl, trifluoromethyl, difluoromethyl, fluoromethyl, chlorodifluoromethyl, dichlorofluoromethyl, methoxy, ethoxy, n- or i-propoxy, trifluoromethoxy or difluoromethoxy.

4. Substituted decalinamines according to Claim 1, where in the formula (I)

$R^1$ represents in each case straight-chain or branched propyl, butyl, pentyl, hexyl, allyl, butenyl, pentenyl, propargyl, butinyl, hydroxyethyl, hydroxypropyl, methoxyethyl, methoxypropyl, ethoxyethyl, ethoxypropyl, hydroxyethoxyethyl, dimethoxyethyl, diethoxyethyl, tetrahydrofuranylmethyl, tetrahydropyranylmethyl, dioxolanylmethyl, dioxolanylethyl, dioxanylmethyl, cyclopropylmethyl, dichlorocyclopropylmethyl, dimethylcyclopropylmethyl, dichlorodimethylcyclopropylmethyl, cyclopentyl, cyclohexyl or cyclohexylmethyl, and

$R^2$ represents hydrogen, methyl, ethyl or in each case optionally straight-chain or branched propyl, butyl, pentyl, hexyl, allyl, butenyl, pentenyl, propargyl, butinyl, hydroxyethyl, hydroxypropyl, methoxyethyl, methoxypropyl, ethoxyethyl, ethoxypropyl, hydroxyethoxyethyl, dimethoxyethyl, diethoxyethyl, tetrahydrofuranylmethyl, tetrahydropyranylmethyl, dioxolanylmethyl, dioxolanylethyl, dioxanylmethyl, dioxanylethyl, cyclopropylmethyl, dimethylcyclopropylmethyl or cyclopentyl, or represents cyclohexyl, cyclohexylmethyl, benzyl, phenylethyl or phenylpropyl which are in each case unsubstituted or substituted once to three times by identical or different substituents comprising methyl or t-butyl, or

$R^1$ and $R^2$ , together with the nitrogen atom to which they are attached, represent a heterocycle of the formula

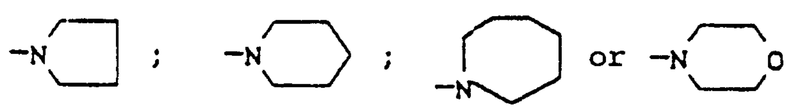

; ; or

which is unsubstituted or substituted once to three times by identical or different substituents, possible substituents in each case being: methyl, ethyl or hydroxymethyl, and

$R^3$ and $R^4$ independently of one another in each case represent hydrogen, methyl, ethyl, n- or i-propyl, t-butyl or trifluoromethyl.

5. Process for the preparation of substituted decalinamines of the general formula (I)

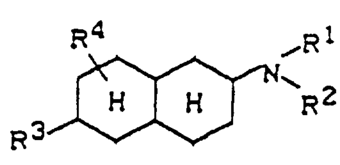

(I)

in which

$R^1$ represents alkyl having more than two carbon atoms, alkenyl, alkinyl, alkoxyalkyl, dialkoxyalkyl, hydroxyalkyl, hydroxyalkoxyalkyl, tetrahydrofuranylalkyl, tetrahydropyranylalkyl, dioxolanylalkyl, dioxanylalkyl or represents in each case unsubstituted or substituted cycloalkylalkyl, cycloalkyl, aralkyl, aralkenyl or aryl,

$R^2$ represents hydrogen, alkyl, alkenyl, alkinyl, alkoxyalkyl, dialkoxyalkyl, hydroxyalkyl, hydroxyalkoxyalkyl, tetrahydrofuranylalkyl, tetrahydropyranylalkyl, dioxolanylalkyl, dioxanylalkyl or represents in each case unsubstituted or substituted cycloalkylalkyl, cycloalkyl, aralkyl, aralkenyl or aryl, or

$R^1$ and $R^2$ , together with the nitrogen atom to which they are attached, represent an unsubstituted or substituted saturated heterocycle which may optionally contain further

heteroatoms, and

$R^3$ and $R^4$ independently of one another each represent hydrogen, alkyl, halogenoalkyl, alkoxy or halogenoalkoxy,

$R^1$ and $R^2$ however, together with the nitrogen atom to which they are attached, only representing an unsubstituted or substituted pyrrolidinyl radical if $R^3$ and $R^4$ do not simultaneously represent hydrogen, and, in the case where $R^4$ represents 9-methyl, $R^3$ does not represent hydrogen, and acid addition salts thereof, characterized in that

(a) decalinones of the formula (II)

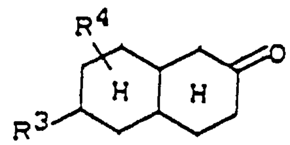

(II)

in which

$R^3$ and $R^4$ have the meaning given above

are reacted with amines of the formula (III)

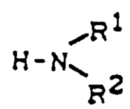

(III)

in which

$R^1$ and $R^2$ have the meaning given above

in the presence of a reducing agent, optionally in the presence of a diluent and optionally in the presence of a reaction auxiliary, or in that

(b) substituted $\beta$-naphthylamines of the formula (IV)

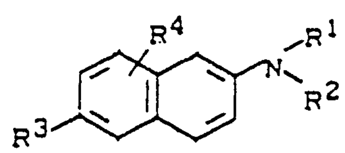

(IV)

in which

$R^1$, $R^2$, $R^3$ and $R^4$ have the meaning given above

are hydrogenated with hydrogen in the presence of a catalyst and optionally in the presence of a diluent;

and then optionally an acid is added.

**6.** Process for the preparation of substituted decalinamines of the formula (Ia)

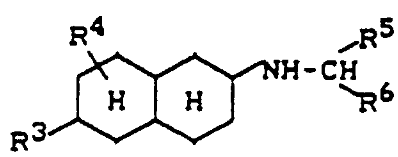

(Ia)

in which

$R^5$ represents alkyl, alkenyl, alkinyl, alkoxy, alkoxyalkyl, dialkoxyalkyl, hydroxyalkyl, hydroxyalkoxyalkyl, tetrahydrofuranyl, tetrahydropyranyl, dioxolanyl, dioxanyl, tetrahydrofuranylalkyl, tetrahydropyranylalkyl, dioxolanylalkyl, dioxanylalkyl or represents in each case unsubstituted or substituted cycloalkylalkyl, cycloalkyl, aralkyl, aralkenyl or aryl,

| | |
|---|---|
| $R^6$ | represents hydrogen or alkyl and |
| $R^3$ and $R^4$ | have the meaning given in Claim 5, |
| $R^5$ | only representing methyl if $R^6$ does not simultaneously represent methyl, |

characterized in that decalinamines of the formula (V)

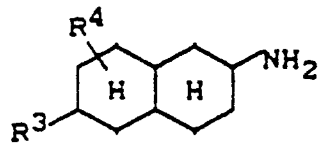

(V)

in which
$R^3$ and $R^4$ have the meaning given in Claim 5 are reacted with aldehydes or ketones of the formula (VI)

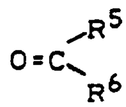

(VI)

in which
$R^5$ and $R^6$ have the meaning given above
in the presence of a reducing agent, optionally in the presence of a diluent and optionally in the presence of a reaction auxiliary, and then optionally an acid is added.

**7.** Process for the preparation of substituted decalinamines of the formula (Ib)

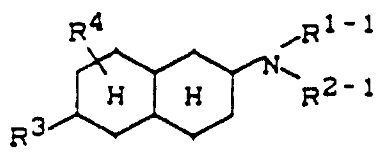

(Ib)

in which

| | |
|---|---|
| $R^{1-1}$ | represents alkyl having more than two carbon atoms, alkenyl, alkinyl, alkoxyalkyl, dialkoxyalkyl, hydroxyalkyl, hydroxyalkoxyalkyl, tetrahydrofuranylalkyl, tetrahydropyranylalkyl, dioxolanylalkyl, dioxanylalkyl, in each case unsubstituted or substituted cycloalkylalkyl, cycloalkyl, aralkyl or aralkenyl, |
| $R^{2-1}$ | represents hydrogen, alkyl, alkenyl, alkinyl, alkoxyalkyl, dialkoxyalkyl, hydroxyalkyl, hydroxyalkoxyalkyl, tetrahydrofuranylalkyl, tetrahydropyranylalkyl, dioxolanylalkyl or dioxanylalkyl, or represents in each case unsubstituted or substituted cycloalkyl, aralkyl, aralkenyl or aryl, and |
| $R^3$ and $R^4$ | have the meaning given in Claim 5, |

characterized in that decalinamines of the formula (VII)

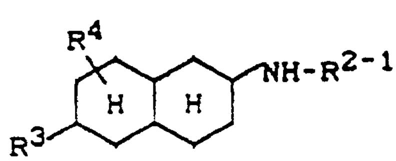

(VII)

in which
$R^{2-1}$, $R^3$ and $R^4$ have the meaning given above
are reacted with alkylating agents of the formula (VIII)

$R^{1-1}$ - E (VIII)

in which

$R^{1-1}$ has the meaning given above and

E represents an electron-attracting leaving group

optionally in the presence of a diluent and optionally in the presence of a reaction auxiliary, and then optionally an acid is added.

**8.** Agent for combating pests, characterized by a content of at least one decalinamine of the formula (I) according to Claims 1 to 7.

**9.** Use of decalinamines of the formula (I) according to Claims 1 to 7 for combating pests.

**10.** Process for combating pests, characterized in that decalinamines of the formula (I) according to Claims 1 to 7 are allowed to act on pests and/or their habitat.

**11.** Process for the preparation of agents for combating pests, characterized in that decalinamines of the formula (I) according to Claims 1 to 7 are mixed with extenders and/or surface-active agents.

**Revendications**

**1.** Décalinamines substituées de formule générale (I)

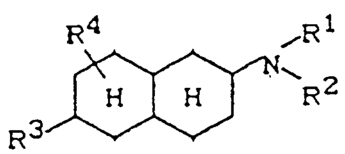

(I)

dans laquelle

$R^1$ est un groupe alkyle ayant plus de deux atomes de carbone, un groupe alcényle, alcynyle, alkoxyalkyle, dialkoxyalkyle, hydroxyalkyle, hydroxyalkoxyalkyle, tétrahydrofurannylalkyle, tétrahydropyrannylalkyle, dioxolannylalkyle, dioxannylalkyle ou un groupe cycloalkylalkyle, cycloalkyle, aralkyle, aralcényle ou aryle, chacun étant non substitué ou substitué,

$R^2$ représente l'hydrogène, un groupe alkyle, alcényle, alcynyle, alkoxyalkyle, dialkoxyalkyle, hydroxyalkyle, hydroxyalkoxyalkyle, tétrahydrofurannylalkyle, tétrahydropyrannylalkyle, dioxolannylalkyle, dioxannylalkyle, ou un groupe cycloalkylalkyle, cycloalkyle, aralkyle, aralcényle ou aryle, chacun étant non substitué ou substitué,

$R^1$ et $R^2$ forment, conjointement avec l'atome d'azote auquel ils sont liés, un hétérocycle saturé non substitué ou substitué qui peut contenir le cas échéant d'autres hétéroatomes et

$R^3$ et $R^4$ représentent chacun, indépendamment l'un de l'autre, l'hydrogène, un groupe alkyle, halogénalkyle, alkoxy ou halogénalkoxy,

$R^1$ et $R^2$ ne pouvant toutefois représenter conjointement avec l'atome d'azote auquel ils sont liés un reste pyrrolidinyle non substitué ou substitué que lorsque $R^3$ et $R^4$ ne représentent pas simultanément de l'hydrogène, et au cas où $R^4$ est un groupe 9-méthyle, $R^3$ n'est pas de l'hydrogène,

ainsi que leur sels d'addition d'acides.

**2.** Décalinamines substituées suivant la revendication 1, dans la formule (I) desquelles

$R^1$ représente un groupe, dans chaque cas linéaire ou ramifié, alkyle ayant 3 à 12 atomes de carbone, alcényle ayant 3 à 8 atomes de carbone, alcynyle ayant 3 à 8 atomes de carbone, hydroxyalkyle ayant 2 à 6 atomes de carbone, alkoxyalkyle ou dialkoxyalkyle avec chacun 1 à 8 atomes de carbone ou hydroxyalkoxyalkyle avec 2 à 6 atomes de carbone dans les parties alkyle individuelles, un groupe, dans chaque cas linéaire ou

ramifié, tétrahydrofurannylalkyle, tétrahydropyrannylalkyle, dioxolannylalkyle ou dioxannylalkyle avec chacun 1 à 4 atomes de carbone dans la partie alkyle ou un groupe cycloalkyle ou cycloalkylalkyle, chacun non substitué ou portant dans la partie cycloalkyle un à plusieurs substituants identiques ou différents, avec chacun 3 à 7 atomes de carbone dans la partie cycloalkyle et 1 à 4 atomes de carbone dans la partie alkyle linéaire ou ramifiée, en considérant dans chaque cas comme substituants : un halogène, un groupe alkyle, alkoxy, halogénalkyle ou halogénalkoxy chacun linéaire ou ramifié avec chacun 1 à 3 atomes de carbone et, le cas échéant, 1 à 9 atomes d'halogènes identiques ou différents ;

ou bien un groupe arylalkyle, arylalcényle ou aryle portant ou non chacun un à plusieurs substituants identiques ou différents dans la partie aryle, avec chacun 6 à 10 atomes de carbone dans la partie aryle et jusqu'à 6 atomes de carbone dans la partie alkyle ou alcényle linéaire ou ramifié, en considérant dans chaque cas comme substituants du groupe aryle : un halogène, un radical cyano, nitro, un radical alkyle, alkoxy, alkylthio, halogénalkyle, halogénalkoxy, halogénalkylthio, alkoxycarbonyle ou alkoximinoalkyle chacun linéaire ou ramifié, avec chacun 1 à 4 atomes de carbone dans les parties alkyle individuelles et le cas échéant 1 à 9 atomes d'halogènes identiques ou différents,

$R^2$ représente l'hydrogène, un groupe, dans chaque cas linéaire ou ramifié, alkyle ayant 1 à 12 atomes de carbone, alcényle ayant 3 à 8 atomes de carbone, alcynyle ayant 3 à 8 atomes de carbone, hydroxyalkyle ayant 2 à 6 atomes de carbone, alkoxyalkyle ou dialkoxyalkyle avec chacun 1 à 8 atomes de carbone ou hydroxyalkoxyalkyle avec 2 à 6 atomes de carbone dans les parties alkyle individuelles, un groupe, dans chaque cas linéaire ou ramifié, tétrahydrofurannylalkyle, tétrahydropyrannylalkyle, dioxolannylalkyle ou dioxannylalkyle avec chacun 1 à 4 atomes de carbone dans la partie alkyle ou un groupe cycloalkyle ou cycloalkylalkyle portant ou non chacun dans la partie cycloalkyle un à plusieurs substituants identiques ou différents, avec chacun 3 à 7 atomes de carbone dans la partie cycloalkyle et 1 à 4 atomes de carbone dans la partie alkyle linéaire ou ramifiée, en considérant dans chaque cas comme substituants : un groupe, linéaire ou ramifié, alkyle, alkoxy, halogénalkyle ou halogénalkoxy avec chacun 1 à 4 atomes de carbone et le cas échéant 1 à 9 atomes d'halogènes identiques ou différents ; ou un groupe arylalkyle, arylalcényle ou aryle, portant ou non chacun un ou plusieurs substituants identiques ou différents dans la partie aryle, avec chacun 6 à 10 atomes de carbone dans la partie aryle et le cas échéant jusqu'à 6 atomes de carbone dans la partie alkyle ou alcényle linéaire ou ramifiée, en considérant comme substituants de la partie aryle : un halogène, un radical cyano, nitro, un radical alkyle, alkoxy, alkylthio, halogénalkyle, halogénalkoxy, halogénalkylthio, alkoxycarbonyle ou alkoximinoalkyle, chacun linéaire ou ramifié avec chacun 1 à 4 atomes de carbone dans les parties alkyle individuelles et le cas échéant 1 à 9 atomes d'halogènes identiques ou différents, ou bien

$R^1$ et $R^2$ forment conjointement avec l'atome d'azote auxquels ils sont liés un hétérocycle pentagonal à heptagonal saturé non substitué ou portant un à plusieurs substituants identiques ou différents, qui peut contenir le cas échéant un autre hétéroatome, et on considère alors comme substituant : un groupe alkyle ou hydroxyalkyle linéaire ou ramifié avec chacun 1 à 4 atomes de carbone et

$R^3$ et $R^4$ représentent chacun indépendamment l'un de l'autre l'hydrogène, chacun un groupe alkyle ou alkoxy linéaire ou ramifié avec chacun 1 à 4 atomes de carbone ou un groupe halogénalkyle ou halogénalkoxy linéaire ou ramifié avec chacun 1 à 4 atomes de carbone et 1 à 9 atomes d'halogènes identiques ou différents.

**3.** Décalinamines substituées suivant la revendication 1, dans la formule (I) desquelles

$R^1$ est un groupe, linéaire ou ramifié, propyle, butyle, pentyle, hexyle, heptyle, octyle, allyle, butényle, pentényle, propargyle, butynyle, hydroxyéthyle, hydroxypropyle, méthoxyéthyle, éthoxyéthyle, propoxyéthyle, butoxyéthyle, méthoxypropyle, éthoxypropyle, propoxypropyle, butoxypropyle, hydroxyéthoxyéthyle, diméthoxyéthyle, diméthoxypropyle, diéthoxyéthyle, un groupe tétrahydrofurannylméthyle, tétrahydropyrannylméthyle, dioxolannylméthyle, dioxolannyléthyle, dioxannylméthyle ou dioxannyléthyle, un groupe cyclopropyle, cyclopropylméthyle, cyclopropyléthyle, cyclopropylpropyle, cyclopentyle,

cyclopentylméthyle, cyclohexyle, cyclohexylméthyle, cyclohexyléthyle ou cyclohexylpropyle portant ou non chacun 1 à 5 substituants, identiques ou différents, méthyle, trifluorométhyle, éthyle, n-propyle, isopropyle, n-butyle, isobutyle, sec.-butyle et/ou tertio-butyle, ou un groupe phényle, benzyle ou phénéthyle portant ou non chacun un à trois substituants identiques ou différents, en considérant alors comme substituants : le fluor, le chlore, le brome, un radical cyano, nitro, méthyle, éthyle, n-propyle, isopropyle, n-butyle, isobutyle, sec.-butyle, tertio-butyle, méthoxy, éthoxy, méthylthio, trifluorométhyle, trifluorométhoxy, trifluorométhylthio, méthoxycarbonyle, éthoxycarbonyle ou méthoximinométhyle et

$R^2$ représente l'hydrogène, un groupe méthyle, éthyle, un groupe, linéaire ou ramifié, propyle, butyle, pentyle, hexyle, heptyle, octyle, allyle, butényle, pentényle, propargyle, butynyle, hydroxyéthyle, hydroxypropyle, méthoxyéthyle, éthoxyéthyle, propoxyéthyle, butoxyéthyle, méthoxypropyle, éthoxypropyle, propoxypropyle, butoxypropyle, hydroxyéthoxyéthyle, diméthoxyéthyle, diméthoxypropyle, diéthoxyéthyle, un groupe tétrahydrofurannylméthyle, tétrahydropyrannylméthyle, dioxolannylméthyle, dioxolannyléthyle, dioxannylméthyle ou dioxannyléthyle, un groupe cyclopropyle, cyclopropylméthyle, cyclopropyléthyle, cyclopropylpropyle, cyclopentyle, cyclopentylméthyle, cyclohexyle ou cyclohexylméthyle portant ou non chacun 1 à 5 substituants, identiques ou différents, méthyle, trifluorométhyle, éthyle, n-propyle, isopropyle, n-butyle, isobutyle, sec.-butyle et/ou tertio-butyle, ou un groupe phényle, benzyle, phényléthyle, phénylpropyle ou phénylbutyle portant ou non chacun 1 à 3 substituants identiques ou différents, en considérant comme substituants dans chaque cas :
le fluor, le chlore, le brome, un radical cyano, nitro, méthyle, éthyle, n-propyle, isopropyle, n-butyle, isobutyle, sec.-butyle, tertio-butyle, méthoxy, éthoxy, méthylthio, trifluorométhyle, trifluorométhoxy, trifluorométhylthio, méthoxycarbonyle, éthoxycarbonyle ou méthoximinométhyle, ou bien

$R^1$ et $R^2$ forment, conjointement avec l'atome d'azote auquel ils sont liés, un hétérocycle non substitué ou portant 1 à 3 substituants identiques ou différents et de formule

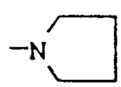 ; 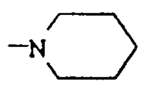 ; 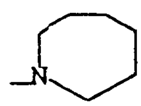 ; 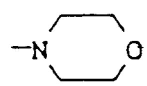

ou 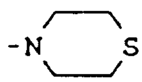

en considérant dans chaque cas comme substituants : un radical méthyle, éthyle ou hydroxyméthyle, et

$R^3$ et $R^4$ représentent chacun, indépendamment l'un de l'autre, l'hydrogène, un groupe méthyle, éthyle, n-propyle, isopropyle, n-butyle, isobutyle, sec.-butyle, tertio-butyle, trifluorométhyle, difluorométhyle, fluorométhyle, chlorodifluorométhyle, dichlorofluorométhyle, méthoxy, éthoxy, n-propoxy, isopropoxy, trifluorométhoxy ou difluorométhoxy.

**4.** Décalinamines substituées suivant la revendication 1, dans la formule (I) desquelles

$R^1$ désigne un groupe, linéaire ou ramifié, propyle, butyle, pentyle, hexyle, allyle, butényle, pentényle, propargyle, butynyle, hydroxyéthyle, hydroxypropyle, méthoxyéthyle, méthoxypropyle, éthoxyéthyle, éthoxypropyle, hydroxyéthoxyéthyle, diméthoxyéthyle, diéthoxyéthyle, tétrahydrofurannylméthyle, tétrahydropyrannylméthyle, dioxolannylméthyle, dioxolannyléthyle, dioxannylméthyle, cyclopropylméthyle, dichlorocyclopropylméthyle, diméthylcyclopropylméthyle, dichlorodiméthylcyclopropylméthyle, cyclopentyle, cyclohexyle ou cyclohexylméthyle et

$R^2$ représente l'hydrogène, un groupe méthyle, éthyle ou un groupe, dans chaque cas éventuellement linéaire ou ramifié, propyle, butyle, pentyle, hexyle, allyle, butényle, pentényle, propargyle, butynyle, hydroxyéthyle, hydroxypropyle, méthoxyéthyle, méthoxypropyle, éthoxyéthyle, éthoxypropyle, hydroxyéthoxyéthyle, diméthoxyéthyle, diéthoxyéthyle, tétrahydrofurannylméthyle, tétrahydropyrannylméthyle, dioxolannylméthyle,

dioxolannyléthyle, dioxannylméthyle, dioxannyléthyle, cyclopropylméthyle, diméthylcyclopropylméthyle, cyclopentyle, un groupe cyclohexyle, cyclohexylméthyle, benzyle, phényléthyle ou phénylpropyle portant ou non dans chaque cas 1 à 3 substituants méthyle ou tertio-butyle identiques ou différents, ou bien

$R^1$ et $R^2$ forment, conjointement avec l'atome d'azote auquel ils sont liés, un hétérocycle non substitué ou portant un à trois substituants, identiques ou différents, et de formule

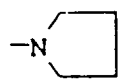 ; 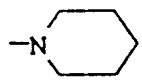 ; 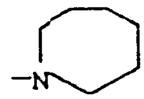 ou 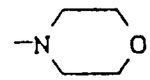

en considérant dans chaque cas comme substituants : un radical méthyle, éthyle ou hydroxyméthyle et

$R^3$ et $R^4$ représentent chacun, indépendamment l'un de l'autre, l'hydrogène, un groupe méthyle, éthyle, n-propyle, isopropyle, tertio-butyle ou trifluorométhyle.

**5.** Procédé de production de décanilamines substituées de formule générale (I)

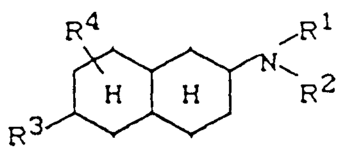

(I)

dans laquelle

$R^1$ est un groupe alkyle ayant plus de deux atomes de carbone, un groupe alcényle, alcynyle, alkoxyalkyle, dialkoxyalkyle, hydroxyalkyle, hydroxyalkoxyalkyle, tétrahydrofurannylalkyle, tétrahydropyrannylalkyle, dioxolannylalkyle, dioxannylalkyle ou un groupe cycloalkylalkyle, cycloalkyle, aralkyle, aralcényle ou aryle, chacun étant non substitué ou substitué,

$R^2$ représente l'hydrogène, un groupe alkyle, alcényle, alcynyle, alkoxyalkyle, dialkoxyalkyle, hydroxyalkyle, hydroxyalkoxyalkyle, tétrahydrofurannylalkyle, tétrahydropyrannylalkyle, dioxolannylalkyle, dioxannylalkyle, ou un groupe cycloalkylalkyle, cycloalkyle, aralkyle, aralcényle ou aryle, chacun étant non substitué ou substitué,

$R^1$ et $R^2$ forment, conjointement avec l'atome d'azote auquel ils sont liés, un hétérocycle saturé non substitué ou substitué qui peut contenir le cas échéant d'autres hétéroatomes et

$R^3$ et $R^4$ représentent, chacun, indépendamment l'un de l'autre, l'hydrogène, un groupe alkyle, halogénalkyle, alkoxy ou halogénalkoxy,

$R^1$ et $R^2$ ne pouvant toutefois représenter conjointement avec l'atome d'azote auquel ils sont liés un reste pyrrolidinyle non substitué ou substitué que lorsque $R^3$ et $R^4$ ne représentent pas simultanément de l'hydrogène, et au cas où $R^4$ est un groupe 9-méthyle, $R^3$ n'est pas de l'hydrogène,

ainsi que leur sels d'addition d'acides, caractérisé en ce que

(a) on fait réagir des décalinones de formule (II)

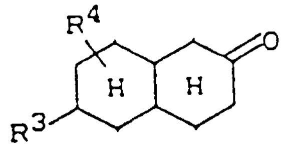

(II)

dans laquelle

$R^3$ et $R^4$ ont la définition indiquée ci-dessus,
avec des amines de formule (III)

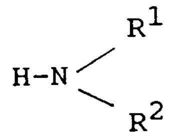

(III)

dans laquelle
$R^1$ et $R^2$ ont la définition indiquée ci-dessus,
en présence d'un agent réducteur, éventuellement en présence d'un diluant et, le cas échéant, en présence d'une substance auxiliaire de réaction, ou bien
(b) on hydrogène des $\beta$-naphtylamines substituées de formule (IV)

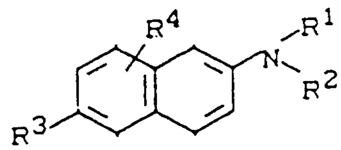

(IV)

dans laquelle
$R^1$, $R^2$, $R^3$ et $R^4$ ont la définition indiquée ci-dessus,
avec de l'hydrogène en présence d'un catalyseur et, le cas échéant, en présence d'un diluant ;
puis le cas échéant, on ajoute un acide.

**6.** Procédé de production de décalinamines substituées de formule (Ia)

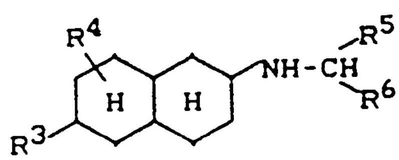

(Ia)

dans laquelle
$R^5$ est un groupe alkyle, alcényle, alcynyle, alkoxy, alkoxyalkyle, dialkoxyalkyle, hydroxyalkyle, hydroxyalkoxyalkyle, tétrahydrofurannyle, tétrahydropyrannyle, dioxolannyle, dioxannyle, un groupe tétrahydrofurannylalkyle, tétrahydropyrannylalkyle, dioxolannylalkyle, dioxannylalkyle ou bien un groupe, non substitué ou substitué, cycloalkylalkyle, cycloalkyle, aralkyle, aralcényle ou aryle,
$R^6$ représente l'hydrogène ou un groupe alkyle et
$R^3$ et $R^4$ ont la définition indiquée dans la revendication 5,
$R^5$ ne représentant un groupe méthyle que lorsque $R^6$ n'est pas en même temps de l'hydrogène,
caractérisé en ce qu'on fait réagir des décanilamines de formule (V)

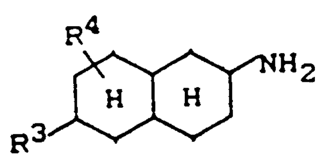

(V)

dans laquelle
$R^3$ et $R^4$ ont la définition indiquée dans la revendication 5,

avec des aldéhydes ou des cétones de formule (VI),

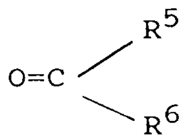

(VI)

dans laquelle

$R^5$ et $R^6$ ont la définition indiquée ci-dessus,

en présence d'un agent réducteur, le cas échéant en présence d'un diluant et en la présence éventuelle d'une substance auxiliaire de réaction, puis on ajoute éventuellement un acide.

**7.** Procédé de production de décanilamines substituées de formule (Ib)

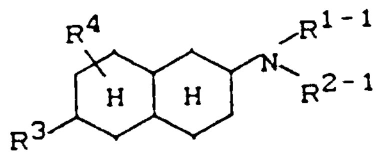

(Ib)

dans laquelle

$R^{1-1}$ est un groupe alkyle ayant plus de deux atomes de carbone, un groupe alcényle, alcynyle, alkoxyalkyle, dialkoxyalkyle, hydroxyalkyle, hydroxyalkoxyalkyle, tétrahydrofurannylalkyle, tétrahydropyrannylalkyle, dioxolannylalkyle, dioxannylalkyle, un groupe, non substitué ou substitué, cycloalkylalkyle, cycloalkyle, aralkyle ou aralcényle,

$R^{2-1}$ représente l'hydrogène, un groupe alkyle, alkyle, alcényle, alcynyle, alkoxyalkyle, dialkoxyalkyle, hydroxyalkyle, hydroxyalkoxyalkyle, tétrahydrofurannylalkyle, tétrahydropyrannylalkyle, dioxolannylalkyle, dioxannylalkyle ou un groupe, non substitué ou substitué, cycloalkyle, aralkyle, aralcényle ou aryle et

$R^3$ et $R^4$ ont la définition indiquée dans la revendication 5,

caractérisé en ce qu'on fait réagir des décanilamines de formule (VII)

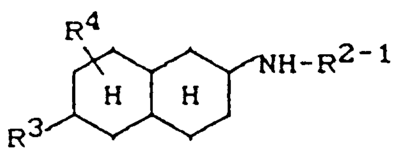

(VII)

dans laquelle

$R^{2-1}$, $R^3$ et $R^4$ ont la définition indiquée ci-dessus,

avec des agents alkylants de formule (VIII)

$R^{1-1}$ - E (VIII)

dans laquelle

$R^{1-1}$ a la définition indiquée ci-dessus et

E est un groupe partant attirant les électrons,

le cas échéant en présence d'un diluant et en la présence éventuelle d'une substance auxiliaire de réaction, puis on ajoute éventuellement un acide.

**8.** Compositions pesticides, caractérisées par une teneur en au moins une décanilamine de formule (I) suivant les revendications 1 à 7.

9. Utilisation de décanilamines de formule (I) suivant les revendications 1 à 7 pour combattre des parasites.

10. Procédé pour combattre des parasites, caractérisé en ce qu'on fait agir des décanilamines de formule (I) suivant les revendications 1 à 7 sur les parasites et/ou sur leur milieu.

11. Procédé de préparation de compositions pesticides, caractérisé en ce qu'on mélange des décanilamines de formule (I) suivant les revendications 1 à 7 avec des diluants et/ou des agents tensio-actifs.